# EUROPEAN PATENT APPLICATION

(11) **EP 2 561 868 A1**
(43) Date of publication of application: **27.02.2013**
(21) Application number: 11178638.0
(22) Date of filing: 24.08.2011
(51) Int. Cl.: A61K 31/12, A61K 31/4706, A61K 45/06, A61P 35/00

(54) **Pharmaceutical compositions comprising hydroxychloroquine (HCQ), Curcumin, Piperine/BioPerine and uses thereof in the medical field**

(71) Applicant: Van Oosten, Anton Bernhard, 3920 Lommel (BE)
(72) Inventor: Van Oosten, Anton Bernhard, 3920 Lommel (BE)
(74) Representative: DeltaPatents B.V.

(57) **Abstract**

The present invention concerns a pharmaceutical composition containing HCQ, curcumin and BioPerine/piperine and its application in the medical field. In particular, the composition according to the invention can be advantageously employed in the prevention or treatment of a subject presenting with a proliferative disorder, to slow the progression of and/or or to cause regression of a premalignant plasma cell proliferative disorder like a monoclonal gammopathy of undetermined significance (MGUS) and/or smoldering (asymptomatic) multiple myeloma (SMM) and/or Indolent multiple myeloma (IMM) and/or to cause remission of a cancer arising from a premalignant plasma cell proliferative disorder like multiple myeloma (MM).

## Description

### FIELD OF THE INVENTION

The invention relates to the field of medicine, pharmaceutical technology and relates to a novel pharmaceutical composition comprising at least a first compound which is an autophagy and/or proteasome and/or Toll like receptor (TLR) modulator and a lysosomotropic agent and at least a second compound which is an autophagy and/or proteasome and/or Toll-like receptor (TLR) modulator and a heat shock protein modulator and a means to increase the water solubility and bioavailability of said second compound and optionally a pharmaceutically acceptable diluent or carrier. More specifically, a pharmaceutical composition of the present invention can be advantageously employed alone or in combination with other agents or therapies for use in the prevention or treatment of and/or to reduce symptoms in a subject presenting with a proliferative disorder, an immunoglobulin (Ig) disorder or an inflammatory disorder or a nervous system disorder or in an asymptomatic subject at risk of developing these disorders. More specifically to slow the progression of and/or to cause regression of a premalignant plasma cell proliferative disorder - a monoclonal gammopathy of undetermined significance (MGUS) and/or smoldering (asymptomatic) multiple myeloma (SMM) and/or Indolent multiple myeloma (IMM) and/or to cause remission of a cancer arising from a premalignant plasma cell proliferative disorder like multiple myeloma (MM).

### BACKGROUND OF THE INVENTION

The plasma cell (proliferative) disorders are monoclonal neoplasms related to each other by virtue of their development from common progenitors in the B lymphocyte lineage. Multiple myeloma, Waldenström's macroglobulinemia, primary amyloidosis, and the heavy chain diseases comprise this group and may be designated by a variety of synonyms such as monoclonal gammopathies, paraproteinemias, plasma cell dyscrasias, and dysproteinemias. Mature B lymphocytes destined to produce Ig bear surface immunoglobulin molecules of both M and D heavy chain isotypes with both isotypes having identical idiotypes (variable regions). Under normal circumstances, maturation to antibody-secreting plasma cells is stimulated by exposure to the antigen for which the surface immunoglobulin is specific; however, in the plasma cell disorders the control over this process is lost. The clinical manifestations of all the plasma cell disorders relate to the expansion of the neoplastic cells, to the secretion of cell products (immunoglobulin molecules or subunits, lymphokines), and to some extent to the host's response to the tumor.

Multiple myeloma (MM) also known as plasma cell myeloma, or myelomatosis or Kahler's disease is a neoplastic process with usually poor prognosis, characterized by bone marrow plasma cell infiltration and production of monoclonal immunoglobulins type G, A, D or E or Bence Jones proteins (κ or λ subunits).

Multiple myeloma may be classified into one of three categories:
Monoclonal gammopathy of undetermined significance (MGUS). This condition is a sort of pre-stage of multiple myeloma, meaning that the excess M protein (monoclonal protein (paraprotein)) is present but the level of plasma cells is low (Bladé and Rosiñol, 2006; Kyle et al. 2010; incorporated herein by reference). It has been established that MM is always preceded by MGUS (Landgren et al., 2009, Weiss et al., 2009, Bladé et al., 2009; Bladé and Rosiñol, 2006; Kyle et al. 2010 incorporated herein by reference). MGUS usually is asymptomatic, but peripheral neuropathy can occur. Although most cases are initially benign, up to 25% (1%/yr) progress to a malignant plasma cell disorder myeloma or a related B-cell disorder, such as macroglobulinemia, amyloidosis, or lymphoma. MGUS occurs in about 1% of the general population and in about 3% of normal individuals over 70 years of age. However, there are no symptoms, other criteria for myeloma diagnosis are absent, and no cause for the increased protein can be identified. Characteristics: Serum M protein (paraprotein) <30 g/L; Bone marrow plasma cells <10%; Absence of anemia, renal failure related to paraprotein, hypercalcemia, lytic bone lesions. Several other illnesses can present with a monoclonal gammopathy, and the monoclonal protein may be the first discovery before a formal diagnosis is made, including but not limited to Multiple myeloma, AIDS, Chronic lymphocytic leukemia, Non-Hodgkin Lymphoma, particularly Splenic marginal zone lymphoma and Lymphoplasmocytic lymphoma, Hepatitis C, Connective tissue disease such as Lupus, Immunosuppression following organ transplantation, Waldenström macroglobulinemia (Kyle RA et al., 2011 incorporated herein by reference) and Guillain-Barre syndrome. In addition to multiple myeloma, MGUS may also progress to Waldenström's macroglobulinemia (McMaster and Caporaso, 2007 incorporated herein by reference), primary amyloidosis, B-cell lymphoma, or chronic lymphocytic leukemia (Landgren and Kyle, 2007; incorporated herein by reference).

The International Myeloma Working Group identified the following risk factors for patients with monoclonal gammopathy of undetermined significance: the amount of monoclonal protein, the type of monoclonal protein, the number of bone marrow plasma cells, and the free light chain ratio.

MGUS/SMM is also associated with non malignant disorders including but not limited to autoimmune disorders like Sjögren's syndrome (SS). Sjögren's syndrome (SS) is a chronic autoimmune disease associated with the production of auto-antibodies and is characterised by a progressive lymphocytic and plasma cell infiltration of the salivary and lachrymal glands, leading to xerostomia and xerophthalmia. SS is predominantly a disease of middle-aged women, while myeloma is a disease of the elderly, with only 2% of cases occurring in patients less than 40 years of age. SS has been recognised to have a high incidence of benign monoclonal gammopathy, although MM is very rare. Most of the monoclonal gammopathies in patients with SS involve the IgM class.

Smoldering (asymptomatic) multiple myeloma (SMM) and Indolent multiple myeloma (IMM) is considered an advanced stage of MGUS (Bladé and Rosiñol, 2006; incorporated herein by reference). Patients with asymptomatic multiple myeloma have a monoclonal protein and slightly increased numbers of plasma cells in the bone marrow. They may have mild anemia and/or a few bone lesions, but do not exhibit the renal failure and frequent infections that characterize active multiple myeloma. In these patients the myeloma is static and may not progress for months or years. Asymptomatic multiple myeloma includes both Smoldering Multiple Myeloma (SMM) and Indolent Multiple Myeloma (IMM). Smoldering Multiple Myeloma (SMM) characteristics; Serum M protein >3 g/L and/or bone marrow plasma cells ≥10%. Absence of anemia, renal failure, hypercalcemia, lytic bone lesions. Indolent Multiple Myeloma (IMM); characteristics, stable serum/urine M protein, bone marrow plasmacytosis, mild anemia or few small lytic bone lesions and absence of symptoms. Disease Management to date involves observation with treatment beginning at disease progression.

Symptomatic Multiple Myeloma (MM). Multiple myeloma is the second most prevalent blood cancer (10%) after non-Hodgkin's lymphoma. It represents approximately 1% of all cancers and 2% of all cancer deaths. Although the peak age of onset of multiple myeloma is 65 to 70 years of age, recent statistics indicate both increasing incidence and earlier age of onset. Patients who present with symptoms typically have a monoclonal protein and increased numbers of plasma cells in the bone marrow. They also have anemia, kidney failure, increased levels of calcium in the blood (hypercalcemia), or bone lesions. Patients with symptomatic myeloma require immediate treatment. Characteristics; presence of serum/urine M protein, bone marrow plasmacytosis (>30%), anemia, renal failure, hypercalcemia, or lytic bone lesions. B lymphocytes start in the bone marrow and move to the lymph nodes. As they progress, they mature and display different proteins on their cell surface. When they are activated to secrete antibodies, they are known as plasma cells. Multiple myeloma develops in B lymphocytes after they have left the part of the lymph node known as the germinal center. The normal cell line most closely associated with MM cells is generally taken to be either an activated memory B cell or the precursor to plasma cells, the plasmablast. A chromosomal translocation between the immunoglobulin heavy chain gene (on the fourteenth chromosome, locus 14q32) and an oncogene (often 11q13, 4p16.3, 6p21, 16q23 and 20q11) is frequently observed in patients with multiple myeloma. This mutation results in dysregulation of the oncogene which is thought to be an important initiating event in the pathogenesis of myeloma. The result is proliferation of a plasma cell clone and genomic instability that leads to further mutations and translocations. The chromosome 14 abnormality is observed in about 50% of all cases of myeloma. Deletion of (parts of) the thirteenth chromosome is also observed in about 50% of cases. Production of cytokines) (especially IL-6) by the plasma cells causes much of their localized damage, such as osteoporosis, and creates a microenvironment in which the malignant cells thrive. Angiogenesis (the attraction of new blood vessels) is increased. The produced antibodies are deposited in various organs, leading to renal failure, polyneuropathy and various other myeloma-associated symptoms. Related conditions include *solitary plasmacytoma* (a single tumor of plasma cells, typically treated with irradiation), *plasma cell dyscrasia* (where only the antibodies produce symptoms, e.g. AL amyloidosis), and POEMS syndrome (peripheral neuropathy, organomegaly, endocrinopathy, monoclonal plasma cell disorder, skin changes).

There is somewhat of an overlap between the various myeloma categories and stages. Most cases of myeloma also feature the production of a paraprotein (monoclonal protein). The paraprotein is produced by the tumor clone. A paraprotein is an abnormal monoclonal immunoglobulin or immunoglobulin light chain (Bence Jones protein) present in blood or urine arising from clonal proliferation of mature B cells, most commonly plasma cells or B-lymphocytes. Alternative terms include monoclonal protein or M-band. Very rarely, the myeloma is *nonsecretory* (not producing immunoglobulins). In theory, multiple myeloma can produce all classes of immunoglobulin, but IgG paraproteins are most common, followed by IgA and IgM. IgD and IgE myeloma are very rare. In addition, light and or heavy chains (the building blocks of antibodies) may be secreted in isolation: κ- or λ-light chains or any of the five types of heavy chains (α-, γ-, δ-, ε- or µ-heavy chains). Additional findings include: a raised calcium (when osteoclasts are breaking down bone, releasing calcium into the bloodstream), raised serum creatinine due to reduced renal function, which may be due to paraprotein deposition in the kidney. A bone marrow biopsy (BMB) is usually performed to estimate the percentage of bone marrow occupied by plasma cells. This percentage is used in the diagnostic criteria for myeloma. Immunohistochemistry (staining particular cell types using antibodies against surface proteins) can detect plasma cells which express immunoglobulin in the cytoplasm but usually not on the surface; myeloma cells are typically CD56, CD38, CD138 positive and CD19 and CD45 negative. Cytogenetics may also be performed in myeloma for prognostic purposes, including a myeloma-specific FISH and Virtual Karyotype. Other useful laboratory tests include quantitative measurement of IgA, IgG, IgM , IgE, IgD (immunoglobulins) to look for immune paresis, and β2-microglobulin which provides prognostic information. On peripheral blood smear the rouleaux formation of red blood cells is commonly seen.

To date MGUS, SMM and MM remains incurable despite recent remarkable advances in therapy. Although no cure exists, nowadays therapies are aimed at preventing or relieving symptoms and complications, destroying abnormal plasma cells, and slowing progression of the disorder. Still the cornerstone of managing multiple myeloma precursor disease involves a prudent "watch and wait" strategy. Outside of clinical trials, there are no current standardized treatment options to date nor drug approved for MGUS or SMM (Neha Korde et al., 2011 - Blood May 26, vol. 117 no. 21 5573-5581 incorporated herein by reference).

### SUMMARY OF THE INVENTION

Multiple myeloma originates from a single clone of plasma cells in the bone marrow. As the neoplastic plasma cells proliferate, bone tissue is destroyed and normal marrow elements are displaced. The neoplastic clone produces a unique immunoglobulin molecule composed of two identical heavy chains and two identical light chains that are covalently linked by disulfide bonds. Because the light chain type produced early in B-cell maturation does not change, it helps to define the clonal nature of the monoclonal process. Normal plasma cells synthesize more light chains than are needed to combine with the heavy chains. These excess unbound light chains pass into the serum as free light chains (FLCs). Due to their relatively small size, 22 kDa for the κ-chain and 44 kDa for the λ-chain, the proteins also clear the kidneys, passing into the glomerular filtrate. The majority of the chains are reabsorbed by the proximal tubules of the kidney, and the rest are excreted into the urine. The small amount of circulating normal polyclonal FLCs causes no damage to the kidneys; however, excessive production of mFLC can lead to formation of β-pleated sheets of AL amyloid, deposition of non-amyloid light chains (light chain deposition disease; LCDD), injury of proximal tubular cells (acquired Fanconi syndrome), or precipitation and obstruction of the distal nephron (cast nephropathy).

In the vast majority of multiple myeloma cases, the clonal plasma cells produce a large M protein - often with excess mFLC - but in about 15% of cases, the M protein is only an FLC. Although detection of the M protein is a key indicator of multiple myeloma, it is important to realize that the disease is a member of a large family of plasma cell proliferative disorders. Multiple myeloma stands out as the most malignant member of the family, but it is far from the most prevalent member of this family of diseases that produce an M protein.

### Progression to Multiple Myeloma: Subcategorizing Risks in MGUS or SMM

MGUS and SMM can remain stable for prolonged periods of time. MGUS and SMM are asymptomatic, and each carries a considerably different potential for progression to multiple myeloma. By definition, patients with MGUS have <3 g/L M protein in serum and <10% of plasma cells in the bone marrow, but they do not have hypercalcemia, anemia, lytic bone lesions, or organ failure. Overall, the risk of MGUS progressing to multiple myeloma or other treatable plasma cell proliferative processes is about 1% per year.

In SMM, the clinical and laboratory findings are the same as MGUS except that the serum M protein is ≥3 g/L and/or the bone marrow plasma cells are ≥10%. The risk of SMM progressing to symptomatic multiple myeloma or AL amyloidosis is about 10% per year for the first 5 years after diagnosis, about 3% per year for the next 5 years, and 1% per year for the succeeding 10 years. The cumulative probability for progression is 73% at 15 years.

The fact that after 10 years of follow-up the risk of progression of SMM falls to 1-2% per year and because of the risk of toxic and even fatal complications of the MM treatments, well described in the art, individuals with early myeloma (smoldering or indolent myeloma, or MGUS) are advised to wait months to years before considering available treatments, despite the fact that a percentage of patients will eventually develop full-blown myeloma (MM). Patients are monitored for progression to myeloma. A complicating factor is that it is frequently difficult to differentiate MM and SMM. Because many organs can be affected by myeloma, the symptoms and signs vary greatly. There is no absolute criterion that helps to differentiate these entities, but an unequivocal presence of complications such as anemia, hypercalcemia, renal failure, or bone disease indicates active MM. However, patients with non-stable asymptomatic myeloma are likely to progress to symptomatic multiple myeloma within 6 to 12 months and require immediate treatment.

Myeloma is still considered incurable, but remissions may be induced with steroids (dexamethasone (Decadron) or prednisone), chemotherapy and stem cell transplants. Newer drugs, immune modulating agents such as thalidomide (Thalomid®), lenalidomide (Revlimid®), and bortezomib (Velcade®) such as lenalidomide and bortezomib, are often used in more advanced disease. Radiation therapy is sometimes used to treat bone lesions that are causing symptoms. The disease develops in 1-4 per 100,000 people per year. It is more common in men, and is twice as common in blacks as it is in whites. With conventional treatment, the prognosis is 3-4 years, which may be extended to 5-7 years with advanced treatments. Multiple myeloma is the second most common hematological malignancy (13%) and constitutes 1% of all cancers. Treatment for multiple myeloma is focused on disease containment and suppression. If the disease is completely asymptomatic (i.e. there is a paraprotein and an abnormal bone marrow population but no end-organ damage), treatment may be deferred. In addition to direct treatment of the plasma cell proliferation, bisphosphonates (e.g. pamidronate or zoledronic acid) are routinely administered to prevent fractures and erythropoietin to treat anemia. Initial treatment of multiple myeloma depends on the patient's age and comorbidities. In recent years, high-dose chemotherapy with hematopoietic stem-cell transplantation has become the preferred treatment for patients under the age of 65. Prior to stem-cell transplantation, these patients receive an initial course of induction chemotherapy. The most common induction regimens used today are thalidomide-dexamethasone, bortezomib based regimens, and lenalidomide-dexamethasone. Autologous stem cell transplantation (ASCT), the transplantation of a patient's own stem cells after chemotherapy, is the most common type of stem cell transplantation for multiple myeloma. It is not curative, but does prolong overall survival. Allogeneic stem cell transplantation, the transplantation of a healthy person's stem cells into the affected patient, has the potential for a cure, but is only available to a small percentage of patients. Furthermore, there is a 5-10% treatment-associated mortality rate.

Stem cell transplantation may not be an option for many people because of advanced age, presence of other serious illness, or other physical limitations. For these patients, the standard of care has been chemotherapy with melphalan and prednisone. Recent studies among this population suggest improved outcomes with new chemotherapy regimens. Treatment with bortezomib, melphalan and prednisone had an estimated overall survival of 83% at 30 months, lenalidomide plus low-dose dexamethasone an 82% survival at 2 years and melphalan, prednisone and lenalidomide had a 90% survival at 2 years. Deep venous thrombosis and pulmonary embolism are the major side effects of thalidomide and lenalidomide. Lenalidomide causes more myelosuppression, and thalidomide causes more sedation. Peripheral neuropathy and thrombocytopenia are major side effects of bortezomib.

In younger patients, post-ASCT maintenance therapy with thalidomide appears to increase tumor burden reduction further, which translates into prolonged PFS (progression free survival). The role of maintenance therapy with thalidomide, lenalidomide, or bortezomib for patients with multiple myeloma is not definitively established. The natural history of myeloma is of relapse following treatment. Depending on the patient's condition, the prior treatment modalities used and the duration of remission, options for relapsed disease include re-treatment with the original agent, use of other agents (such as melphalan, cyclophosphamide, thalidomide or dexamethasone, alone or in combination), and a second autologous stem cell transplant.

Later in the course of the disease, "treatment resistance" occurs. This may be a reversible effect, and some new treatment modalities may re-sensitize the tumor to standard therapy. For patients with relapsed disease, bortezomib (or Velcade) is a recent addition to the therapeutic arsenal. Bortezomib is a proteasome inhibitor. Finally, lenalidomide (or Revlimid), a less toxic thalidomide analog, is showing promise for treating myeloma. Renal failure in multiple myeloma can be acute (reversible) or chronic (irreversible). Acute renal failure typically resolves when the calcium and paraprotein levels are brought under control. Treatment of chronic renal failure is dependent on the type of renal failure and may involve dialysis.

Because current therapy is rarely curative, most people go through many risky and potentially health damaging treatment regimens during the course of their illness. Recently, the number of effective treatments has increased, and as a result, the average survival has nearly doubled. But survival time varies widely depending on certain features, such as kidney problems, blood levels of certain proteins including beta₂-microglobulin and serum albumin, and genetic characteristics, at the time of diagnosis and the response to treatment. Occasionally, people who survive for many years after successful treatment of multiple myeloma develop leukemia or irreversible loss of bone marrow function. These late complications may result from chemotherapy and often lead to severe anemia and an increased susceptibility to infections and bleeding.

Recent trials are using an off-patent drug called hydroxychloroquine (HCQ), an antimalarial drug, sold under the trade names *Plaquenil, Dolquine, and Quensyl,* is already used to treat several conditions, including malaria and rheumatoid arthritis. Hydroxychloroquine (HCQ) is an immunosuppressive lysosomotropic amine. Hydroxychloroquine (HCQ) improves dry eye symptoms of patients with primary Sjogren's syndrome. HCQ has three known applications: anti-malaria, calming the immune system and inhibition of autophagy. Normal cells rely on autophagy to maintain a balance between the synthesis and degradation of proteins and organelles during development or in times of stress. Cancer cells rely on this process as well, not just to survive in the inhospitable environment of a tumor, but also to ward off the effects of chemotherapy and radiation.

HCQ is now being tested/combined with a number of therapies as an autophagy inhibitor. Autophagy is a necessary step for cell to resist cytotoxicity. HCQ has been combined with anti-multiple myeloma chemotherapeutic agents doxorubicine, epidoxorubicin, cis-platinum and mitoxantrone and shown to act as an autophagy inhibitor in *in-vitro* cultured cell lines [CN101428025; incorporated herein as a reference]. As a potential therapeutic target, the autophagy pathway has significant promise, but it has potential pitfalls. Autophagy, for example, appears to have the ability to prevent healthy cells from becoming cancerous, by allowing them to degrade junk that might otherwise induce DNA damage and drive a cell toward malignancy. The same pathway also helps the immune system recognize potential threats, so disrupting it could, in theory, inhibit the body's ability to mount an immune response against cancer.

Natural agents such as curcumin may have activity as a cancer chemopreventive and chemotherapeutic agent [for extensive review -Teiten et al., 2010; Toxins, 2, 128-162, incorporated herein by reference]. However it appears that many of the anti-cancer effects of curcumin observed *in vitro* cannot be achieved *in vivo* or in patients mainly due to its low bioavailability outside the gastrointestinal tract after oral administration.

Piperine is the alkaloid responsible for the pungency of black pepper and long pepper, along with chavicine (an isomer of piperine). Piperine has also been found to inhibit human CYP3A4 and P-glycoprotein, enzymes important for the metabolism and transport of xenobiotics and metabolites. In animal studies, piperine also inhibited other enzymes important in drug metabolism. By inhibiting drug metabolism, piperine may increase the bioavailability of various compounds and alter the effectiveness of some medications. Notably, piperine may enhance bioavailability of curcumin by 2000% in humans (Shoba et al., 1998; Planta Med. 64 (4): 353-6. incorporated herein by reference) and can also act as an anticancer agent alone or in combination with HCQ and/or curcumin. The invention includes any compound comprising a piperonyl group for use in combination with HCQ and/or curcumin in the prevention or treatment of a proliferative disorder and/or a premalignant proliferative disorder. The invention further includes any compound comprising a piperonyl group in combination with HCQ and/or curcumin for use to slow the progression of and/or or to cause regression of a premalignant plasma cell proliferative disorder like a monoclonal gammopathy of undetermined significance (MGUS) and/or smoldering (asymptomatic) multiple myeloma (SMM) and/or Indolent multiple myeloma (IMM) and/or to cause remission of a cancer arising from a premalignant plasma cell proliferative disorder like multiple myeloma (MM).

Potential risks and side-effects of curcumin:- (Kawanishi et al., 2005; incorporated herein by reference) remarked that curcumin, like many antioxidants, can be a "double-edged sword" where in the test tube, anti-cancer and antioxidant effects may be seen in addition to pro-oxidant effects. Carcinogenic effects are inferred from interference with the p53 tumor suppressor pathway, an important factor in human colon cancer. Carcinogenic and LD50 tests in mice and rats, however, have failed to establish a relationship between tumorigenesis and administration of curcumin in turmeric oleoresin at >98% concentrations. In animal studies, hair loss (alopecia) and lowering of blood pressure have been reported. Clinical studies in humans with high doses (2-12 grams) of curcumin have shown few side effects, with some subjects reporting mild nausea or diarrhea. More recently, curcumin was found to alter iron metabolism by chelating iron and suppressing the protein hepcidin, potentially causing iron deficiency in susceptible patients.

Several recent lab studies in myeloma cells and mice have demonstrated that curcumin kills myeloma cells and increases the effects of conventional drug therapy. They found that curcumin reduces the production of a protein that helps tumor cells repair damaged DNA. Curcumin slowed down the growth of multiple myeloma cells that are resistant to dexamethasone (Decadron), doxorubicin (Adriamycin), and melphalan. It also enhanced the effects of thalidomide (Thalomid) and Velcade (bortezomib). By performing experiments on mice, the researchers confirmed curcumin's ability to augment the effect of Velcade.

In early stage clinical trials conducted studying curcumin's effects in patients with multiple myeloma and MGUS, no objective responses were noted. From a clinicians point of view the decision not to treat MGUS or SMM is the unpredictability of the progression of the disease to MM, the negative side effects of the treatments, resistance to the treatments and so on. From a patient's point of view however the real risks of progression of the disease makes it imperative that an innovative treatment to prevent the early/precursor stages of the myeloma MGUS/SMM progressing to MM is found. Overall the risk of progression of MGUS to myeloma or related disorder is 1% per year. Although the prevalence of MGUS increases with advancing age, after adjustment for the level of the M-protein, the annual risk of progression to myeloma or a related cancer is not affected by age or the duration of MGUS. Younger patients are more likely to have progression to cancer during their lifetime because they are at risk for longer. It is currently not possible to predict the course in any individual patient, and clinically symptomatic myeloma may not evolve for as long as 20 years. Parameters that are currently used to identify those patients at highest risk of developing disease progression are as follows: the size of the M-protein, the type of M-protein with IgA and IgM paraproteins having a higher risk compared with IgG paraproteins, the percentage of bone marrow plasma cells, and an abnormal serum-free light chain ratio. Changes in the level of the serum paraprotein are regularly used to form the basis of assessing the response to therapy and monitoring the progress of the disease. However, studies suggest that although discrimination levels of serum paraprotein are generally used to distinguish between asymptomatic and symptomatic monoclonal gammopathies, the serum MC concentrations higher than the given cutoffs may remain stable for prolonged periods. Recent studies have shown that the test for the paraprotein may not be an exact science. The presence of large amounts of monoclonal paraprotein in the serum can lead to a range of spurious laboratory results. These may result from analytical interference of the M-protein through chemical or immunological means, or from pre-analytical interference [King and Florkowski, 2010; Pathology: August 2010 - Volume 42 - Issue 5 - p 397-40].

In addition a higher risk of evolution to MM was detected in SMM patients compared with MGUS patients (HR, 3.1; 95% CI, 1.9 to 4.9; P .0001), and this parameter was collinear with the percentage ofBMPCs. The upper limit of safety of BMPCs has been suggested at 5%, 10%, or 20% by different studies. Baldini et al., 1996; blood 1996 87: 912-918, incorporated herein by reference has shown the importance of 10% as the upper limit for a diagnosis of benign gammopathy in IgG patients, higher percentages being significantly associated with a worse clinical outcome. The number of progression incidences or "events" is significantly higher for SMM patients with BMPC>10% than with BMPC≤10% according to Cesana et al., 2002; Journal of Clinical Oncology, Vol 20, No 6: pp 1625-1634; incorporated herein by reference [Table 4].

To date there are no agents known that are curative for either smoldering myeloma (SMM) or symptomatic multiple myeloma (MM). The agents that that have been found for treatment of myeloma have undesirable side effects are generally costly, and there is no evidence that they can eliminate the plasma cell clone. Current agents for the treatment of multiple myeloma (MM) can reduce the M-spike in patients with smoldering myeloma (SMM), but it is not known if this can actually prolong the time to the development of symptomatic myeloma. Studies have shown that although the disorders MGUS and SMM represent clonal proliferation of plasma cells, they do not behave like malignancies [Rajkumar SV et al. 2010; incorporated herein by reference].

In common with all cancers, the plasma cells in myeloma are identical ('clonal'), because they originate from a single abnormal cell that starts to multiply out of control. The protein produced is, therefore, also identical ('monoclonal' meaning the product of a single clone). So any early intervention with the aim of elimination the plasma cell clone not just the paraprotein load would be the most desired innovative therapeutic tool. Current drugs that are used for multiple myeloma will reduce the paraprotein (M-protein) but the monoclonal clone of plasma cells and plasma cell proliferation so far cannot be readily reduced nor eliminated. Furthermore current monitoring techniques such as using the size of the M protein as a risk factor may be not accurate enough. Increased/decreased Ig production rate per cell is not directly linked to increase/decrease in plasma cell generation (Morimoto S et al., 2000: incorporated herein by reference).

A number of HCQ clinical trials testing autophagy inhibition are actively recruiting patients with a variety of cancers, including breast, colorectal, myeloma, and chronic lymphocytic leukemia. Many of the HCQ trials have reported severe side effects with using HCQ (at the standard dose tested 200 mg) in combination with other compounds like the proteasome inhibitor bortezomib (Velcade®)) and no significant benefits from the combination were observed to warrant routine use [(Vogel T et al., 2008; T Vogel et al., Oct 26th 2010; 50th ASH meeting and exposition; incorporated herein by reference]. Several cancer treatment approaches, such as HSP90 inhibitors (geldanamycin) and proteasome inhibitors (Bortezomib), may induce a proteotoxic stress that activates a pro-survival pathway, which could explain the low efficiency of these therapies in clinical practice.

Surprisingly we found that HCQ at the standard dose 200mg in combination with curcumin and piperine/BioPerine was significantly capable of reducing/eliminating the plasma cell clone (BMPCs) in a premalignant plasma cell proliferative disorder SMM without any observable side effects causing disease regression for SMM to near MGUS. Furthermore the results as disclosed herein the invention give the first *in-vivo* confirmation to show that that monoclonal IgG paraprotein secretion is not a reliable indicator of disease status. Large amounts of monoclonal paraprotein in the serum can lead to a range of spurious laboratory results. These may result from analytical interference of the M-protein through chemical or immunological means, or from pre-analytical interference.

A pharmaceutical composition as disclosed herein the invention unexpectedly can substantially reduce/eliminate the plasma cell clone (BMPCs) in a premalignant plasma cell proliferative disorder without substantially altering paraprotein levels and in doing so surprisingly gives rise to a regression of a plasma cell disorder more in particular a plasma cell myeloma from a near MM state to SMM to MGUS (i.e. limited and regulated forms of plasma cell proliferation) *in-vivo* without any undesirable side effects and as a result a much more beneficial clinical outcome for the patient. A higher bone marrow plasma cell concentration (BMPC) is significantly associated with a worse clinical outcome (table 4). According to Korde *et al., 2011:* "At this time, no drugs have been approved for SMM Although it requires the launching of larger studies and with longer follow-up, it seems reasonable to argue that future approvals of drugs for SMM should based on overall survival advantages, and that progression-free survival is not adequate" [Korde et al., 2011: Blood May 26, 2011 vol. 117 no. 21 5573-5581 incorporated herein by reference].

The chance of a progression from SMM to MM is much greater than from MGUS to MM. Remission of cancer is a rare event with a reported incidence of less than one in 60,000 to 100,000 people with cancer.

### DETAILED DESCRIPTION OF THE INVENTION

It is an object of the present invention to provide a pharmaceutical composition according to the invention comprising at least a first compound (one or more) which is an autophagy and/or proteasome and/or Toll like receptor (TLR) modulator and a lysosomotropic agent, and/or at least a second compound (one or more) which is an autophagy and/or proteasome and/or Toll-like receptor (TLR) modulator and a heat shock protein modulator and optionally a means to increase the water solubility and bioavailability of said second compound and optionally a pharmaceutically acceptable diluent or carrier.

Even more preferred it is an object of the present invention to provide pharmaceutical composition comprising at least a first compound (one or more) which is an autophagy and/or proteasome and/or Toll like receptor (TLR) modulator and a lysosomotropic agent and at least a second compound (one or more) which is an autophagy and/or proteasome and/or Toll-like receptor (TLR) modulator and a heat shock protein modulator and optionally a means to increase the water solubility and bioavailability of said second compound and optionally a pharmaceutically acceptable diluent or carrier.

Preferably said heat shock protein modulator is a heat shock protein 90 and/or 70 (Hsp90 and/or Hsp70) modulator and can modulate the heat shock protein 90 (Hsp90 and/or Hsp70) response/function even under none or transient - (cellular) stress conditions. By modulating Hsp90 and/or Hsp 70 function as used herein means to decrease Hsp90 and/or Hsp70 activity. Preferably to disrupt Hsp90 and/or Hsp70 function directly or indirectly, for example said modulator of Hsp90 and/or Hsp70 has Hsp90 and/or Hsp70 inhibitor activity.

Preferably said autophagy modulators are autophagy inhibitors [For review of autophagy see Klionsky et al., 2008a; Mizushima, 2004; Mizushima and Yoshimori, 2007; Rubinsztein et al., 2009; the contents of which are incorporated herein by reference].

Preferably said first compound which is a Toll like receptor modulator is a Toll like receptor 9 (TLR-9) inhibitor. Preferably said second compound which is a Toll like receptor modulator is a Toll like receptor 4 (TLR-4) inhibitor. Toll-like receptor (TLR) family are known in the art to play a fundamental role in pathogen recognition and activation of innate immunity [for review see Lien E, Ingalls RR (2002). "Toll-like receptors." Crit. Care Med. 30 (1 Suppl): S1-11, included herein by reference].

A proteasome as used herein can be an immunoproteasome and the like. Preferably said proteasome modulators are proteasome inhibitors. A lysosomotropic agent as used herein may cause direct destabilization of lysosomes, lysosome leakage, incomplete lysosome-autophagosome fusion and dysfunction triggering cell death. For example, said lysosomotropic agent is a lysosomotropic amine or the like which accumulates preferentially in the lysosomes of cells in the body.

Poor water solubility and short biological half-life of a compound can be improved by methods known in the art including the use of with stabilizing agents like (pharmaceutic) adjuvants, the formulation of delivery vehicles consisting of liposomes, nanoparticules, comprising pharmaceutical compositions of the present invention and the like. For example but not limited to said means can be the addition of at least one compound, a third compound to the pharmaceutical composition which is an alkaloid preferably an alkaloid from a pyridine-piperidine group of alkaloids.

It is a further object of the present invention to provide a pharmaceutical composition according to the invention comprising at least a first compound (one or more) which is a non-competitive proteasome inhibitor and a lysosomotropic agent and/or a second compound (one or more) which is a non-competitive proteasome inhibitor which can modulate Hsp 90 function.

Preferably said first and second compounds according to the invention are non-competitive (indirect) proteasome inhibitors. A non competitive inhibitor as used herein is a molecule which binds away from the catalytic centers, yet is able to modifying the performance of the enzyme/proteasome in a negative manner. For example a natural or synthetic compound which does not specifically targeting catalytic sites of the enzyme/proteasome (act on sites of the proteasome outside of the catalytic center) but has a negative effect on the action of the enzyme/proteasome.

Preferably said second compound which is a proteasome modulator is a non-competitive proteasome inhibitor which can simultaneously modulate heat shock protein, preferably Hsp90 and/or proteasome function. Even more preferred said second compound can act as a non-competitive proteasome inhibitor and modulate the heat shock protein 90 response/function even under none or transient - (cellular) stress conditions. By modulating Hsp90 function as used herein means to decrease Hsp90 activity. Preferably to disrupt Hsp90 function directly or indirectly, for example said modulator of Hsp90 has Hsp90 inhibitor activity. For example but not limited to Hsp90 inhibitory activity can be affected through down-regulating/depletion of Hsp90 client proteins, more particularly but not limited to Raf-1, survivin, Cdk4, Akt, EGFR and the like [Pearl and Prodromou 2006; Annual Review of Biochemistry Vol. 75: 271-294 incorporated herein by reference].

It is a further object of the present invention to provide a pharmaceutical composition according to the invention, whereby said first compound is a quinolinic compound or quinolinic - like compound (one or more) or a pharmaceutically acceptable salt thereof and whereby said second compound is a curcuminoid or curcuminoid like-compound (one or more) or a pharmaceutically acceptable salt thereof and whereby said means to increase the water solubility and bioavailability of said second compound is the addition of at least one compound from a pyridine-piperidine group of alkaloids or a pharmaceutically acceptable salt thereof and optionally a pharmaceutically acceptable diluent or carrier.

Included are (natural/synthetic) analogs, conjugates, derivatives, or isomers of said compounds. Analogs, conjugates, derivatives, or isomers of said compounds are well known in the art and are encompassed within the invention.

Examples of quinolinic compounds or quinolinic like compound are well known in the art and are encompassed herein. Included are (natural/synthetic) derivatives, isomers (see US55314894 incorporated herein by reference; Stecher, VJ and Michner WF, 1997) and analogs/analogues and salts of quinolinic compound or quinolinic like compounds.

A quinolinic (like) compound is a compound comprising a quinolinic structure/core/unit. Preferably said quinolinic like compound is one which has a mode of action or activity like a quinolinic compound as disclosed herein more in particular the ability to act alone or in combination with a curcuminoid or curcuminoid like compound and/or a pyridine-piperidine group of alkaloids similar to/like a quinolinic compound to reduce/eliminate the abnormal cell clone of a proliferative disorder. An abnormality in the production of any of the mature blood cells or their precursors (immature forms) can give rise to the abnormal cell clone with abnormal growth and differentiation. By way of example the overproduction of one type of cell, or one cell line may become predominant because the cells don't die at a normal rate.

For example said quinolinic like compound is a tripyrrolic compound/molecule for example but not limited to prodigiosin (prodiginines/prodigiosines) and the like. Also included but not limited to lumefantrine (alone or combined with artemether), halofantrine, antifolates, DHFR inhibitors (antifols), 2,4-Diaminopyrimidines (Pyrimethamine), Diaminodiphenysulfone or dapsone, biguanides (Proguanil, Chlorproguanil); Sulfonamides, Sulfadoxine, Sulfalene, Sesquiterpene lactones, artemether, artesunate, Dihydroartemisinin, Arteether/Artemotil, Artemisinin; Tovaquone (always combined with proguanil as Malarone), Tetracycline, Doxycycline, Clindamycin and the like.

A curcuminoid is a curcumin an analog, isomer or a derivative thereof. A curcuminoid compound as used herein is preferably a component of turmeric, derived from curcuma for example Curcumin (commercial grade curcumin, pure curcumin and the like), analogs of curcumin (Cur), such as demethoxycurcumin (DMC), bisdemethoxycurcumin (BDMC), tetrahydrocurcumin (THC) and the like and derivative thereof. The derivatives are usually more stable than curcumin against hydrolysis in cyclodextrin solution. Included are curcumin extracts, curcumin derivatives, or analogs of curcumin with different chemical groups that have been formed to increase solubility of the curcumin to make it more suitable for drug formulation.

For example but not limited to said curcuminoid is a curcumin ester comprising the esterification product of curcumin and a monocarboxylic like docosahexaenoic acid. Preferably said curcuminoid is selected from the group consisting of curcumin, tetrahydrocurcumin, dihydroxytetrahydrocurcumin, demethoxycurcumin, bisdemethoxycurcumin, diketone, curcumin esters, digalactoside of curcumin, ethylferulate, new complex curcuminoids (cassumunin A and cassumunin B), turmerones and the like. Included are mixtures thereof conjugated curcuminoids, and derivatives of curcuminoids.

A wide variety of other curcumin analogues are known in the art. Representative examples of curcumin analogues are compounds such as: (a) ferulic acid, i.e., 4-hydroxy-3-methoxycinnamic acid and related compounds such as 3,4-methylenedioxy cinnamic acid and 3,4-dimethoxycinnamic acid ; (b) aromatic ketones, such as 4-(4-hydroxy-3methoxyphenyl)-3-buten-2-one, zingerone , 4-(3,4-methylenedioxyphenyl)-2-butanone, 4-(p-hydroxyphenyl)-3-buten-2-one, 4-hydroxyvalerophenone, 4-hydroxybenzylactone, 4-hydroxybenzophenone, 1,5-bis(4-dimethylaminophenyl)-1,4-pentadien- 3-one; (c) aromatic diketones such as 6-hydroxydibenzoylmethane; (d) caffeic acid compounds such as 3,4-dihydroxycinnamic acid; (e) cinnamic acid; (f) aromatic carboxylic acids, such as 3,4-dihydroxyhydrocinnamic acid, 2-hydroxycinnamic acid, 3-hydroxycinnamic acid and 4-hydroxycinnamic acid; (g) aromatic ketocarboxylic acids such as 4-hydroxyphenylpyruvic acid; (h) aromatic alcohols such as 4- hydroxyphenethyl alcohol and the like. For example see Preetha Anand et al., 2008: Biochemical pharmacology 76, 1590-1611, included herein by reference. Included are also water soluble forms of curcumin, acetates and amino acid conjugates.

A curcuminoid like-compound is a compound which has the same or similar activity as curcumin, which is at least capable of interacting with at least one a compound which is an autophagy inhibitor for example a quinolinic compound or like compound to reduce and/or eliminate the plasma cell clone, more specifically pre-malignant proliferating plasma cells/lymphoplasmacytic cells and the like for e.g. abnormal bone marrow plasma cells and the like. For example, a member of the capsaicinoids family of compounds. Preferably said curcuminoid-like compound is a water soluble amino acid conjugates of curcumin [see Sheng Biao Wan et al., 2010, International journal of molecular medicine, Volume: 26, Pages 447-55, incorporated herein by reference]. A curcumoid like compound maybe as for an example a compound which belongs to the capsaicinoids family of substances to which curcumin belongs.

Also included are (natural/synthetic) derivatives, isomers and analogues and salts of said curcuminoid compound or like compound. Examples of such are known in the art [Preetha Anand et al., 2008: Biochemical pharmacology 76, 1590-1611; Kapor et al. 2007, Journal of scientific and industrial research, page 647-650; incorporated herein by reference].

Pyridine-piperidine group of alkaloids are well known in the art for example see Marilyn J. Schneider Alkaloids: Chemical and Biological Perspectives; Volume 10, 1996, Pages 155-299 incorporated herein by reference. Also included are compounds which have piperine like activities like furanocoumarins, silybinin, Itraconazole, clarithromycin, cyclosporine, diltiazem, erythromycin, grapefruit juice, itraconazole, ketoconazole, verapamil, xanthene food dyes - rose bengal (RB), phroxine (PL), amaranth, erythrosine B (ET), allura red, new coccine, acid red (AR), tartrazine, sunset yellow FCF, brilliant blue FCF, and indigo carmine and the like which are able to improve the water solubility and bioavailability of a non- competitive proteasome inhibitor more in particular a curcuminoid compound or like compound.

Preferably said compound from a pyridine-piperidine group of alkaloids is a modulator of NF-κB activation. Preferably said compound from a pyridine-piperidine group of alkaloids inhibits and/or reduces and/or supresses NF-κB activation. Preferably said compound from a pyridine-piperidine group of alkaloids is piperine/BioPerine or an isomer thereof. It has been shown that piperine can dramatically increase absorption of selenium, vitamin B, beta-carotene and curcumin as well as other nutrients. Piperine present in black pepper acts as a thermogenic compound. Piperine enhances the thermogenesis of lipid and accelerates energy metabolism in the body and also increases the serotonin and beta-endorphin production in the brain.

Preferably said first and/or second and/or third compound of the pharmaceutical composition as disclosed herein are capable of interfering with the activity of the transcription factor NF-κB (nuclear factor kappa-light-chain-enhancer of activated B cells). Preferably said first and/or second and/or third compound as disclosed herein can down-regulate NF-κB and or inhibit NF-κB.

Preferably said first and/or second and/or third compound as disclosed herein can alone or in combination suppress angiogenesis for example by causing G2/M cell cycle arrest.

Preferably said curcuminoid or curcuminoid like compound is a proteasome inhibitor. Even more preferrred said second compound is a non-competitive (indirect) proteasome inhibitor. A non competitive inhibitor as used herein is a molecule which binds away from the catalytic centers, yet is able to modifying the performance of the enzyme/proteasome in a negative manner. For example a natural or synthetic compound which does not specifically targeting catalytic sites of the enzyme/proteasome, but has a negative effect on the action of the enzyme. By way of example a curcuminoid or curcuminoid like compound which is an allosteric inhibitor with antiangiogenic activity, preferably a negative allosteric modulator of angiogenesis. Mechanisms of allosteric inhibition are known in the art (see Maria Gaczynska and Pawel A. Osmulski et al., 2005; Methods in Enzymology; Volume 398, pages 425-438; incorporated herein by reference). Preferably said curcuminoid or curcuminoid like compound causes inhibition of the NF-κB (nuclear factor kappa-light-chain-enhancer of activated B cells) signalling pathway and induces apoptosis.

Preferably said curcuminoid or curcuminoid like compound has also anti-immune and/or anti-inflammatory and/or anti-oxidant activity and/or which is capable of interfering with the activity of the transcription factor NF-κB (nuclear factor kappa-light-chain-enhancer of activated B cells).

Preferably the water solubility and bioavailability of said curcuminoid or curcuminoid like compound is enhanced using water soluble water soluble amino acid conjugates of curcumin or analogs of curcumin such as curcumin acetates. Poor water solubility and short biological half-life can also be improved by methods known in the art including the combination of curcumin with adjuvants (e.g. BioPerine/piperine, quercetin, genistein) and the formulation of curcumin in delivery vehicles consisting of liposomes, nanoparticules, phospholipid formulations of curcumin and the like.

It is a further object of the present invention to provide a pharmaceutical composition according to the invention whereby said first compound is an aminoquinolone/aminoquinoline, an analog, derivative, or isomer thereof.

For example but not limited to:
1. Aminoquinolines:
   a. (4-) : amodiaquine (histamine N-methyltransferase inhibitor) the lysosomotropic agents hydroxychloroquine, chloroquine and the like [US 6, 479,504; incorporated herein by reference].
   b. (8-) : primaquine, tafenoquine, pamaquine.
2. 4-methanolquinolines:
   Mefloquine, Quinine, Quinidine.
3. Bisquinoline: Piperaquine [US 2002/0151564, incorporated herein by reference]

It is a further object of the present invention to provide a pharmaceutical composition according to the invention whereby said first compound is selected from the group consisting of Hydroxychloroquine, Chloroquine, Amodiaquine, Primaquine, Tafenoquine, Pamaquine, Mefloquine, Quinine, Quinidine, Piperaquine, and Bisquinoline - analogs, derivatives, isomers and combinations thereof.

It is a further object of the present invention to provide a pharmaceutical composition according to the invention whereby said first compound is hydroxychloroquine and/or Chloroquine and/or Amodiaquine and/or Primaquine and/or Tafenoquine and/or Pamaquine and/or Mefloquine, Quinine and/or Quinidine and/or Piperaquine and/or Bisquinoline their analog, derivatives, isomers thereof.

Even more preferred said first compound is a (4)-aminoquinoline - Hydroxychloroquine (HQ) and/or Chloroquine and/or Amodiaquine analog or derivative thereof. Even more preferred a lysosomotropic (monobasic) amine like HCQ or a Hydroxychloroquine (HQ) like compound. A Hydroxychloroquine (HQ) like compound that as used herein is any salt, compound, isomer, intermediate, derivative, or preparation thereof which is chemically equivalent or identical to Hydroxychloroquine (HQ), which has the same or similar activity as HCQ, at least capable of interacting with at least one compound which is a proteasome inhibitor and an Hsp90 modulator, more specifically a curcuminoid or curcuminoid like-compound, even more preferred curcumin and/or at least one compound from a pyridine-piperidine group of alkaloids to reduce and/or eliminate the abnormal plasma cell clone of a plasma cell proliferative disorder, preferably a premalignant plasma cell proliferative disorder more in particular SMM or IMM or MGUS and the like.

Also included is the use of specific enantiomers (e.g. R and S - enantiomers) of said compounds, for example hydroxychloroquine. Analog as used herein is structural derivative of the parent compound and encompasses a chemical analog, a synthetic analog and the like. The meaning analog is well known in the art.

The invention further provides a pharmaceutical composition according to the invention whereby said first compound is Hydroxychloroquine, said second compound is curcumin and whereby said means to increase the increase the water solubility and bioavailability of said second compound is the addition of a third compound piperine/BioPerine.

The invention further provides a pharmaceutical composition comprising at least one first compound which is an autophagy and/or proteasome and/or Toll like receptor (TLR) modulator and a lysosomotropic agent and at least one second compound which is an autophagy and/or proteasome and/or Toll-like receptor (TLR) modulator and a heat shock protein modulator, and optionally a means to increase the water solubility and bioavailability of said second compound for use as a medicament.

Preferably the invention provides a pharmaceutical composition comprising a first compound (one or more) which is a quinolinic compound or quinolinic like compound or a pharmaceutically acceptable salt thereof and a second compound (one or more) which is a curcuminoid or curcuminoid like-compound or a pharmaceutically acceptable salt thereof for use as a medicament.

Even more preferred the invention provides a pharmaceutical composition comprising a first compound which is hydroxychloroquine (HCQ), and a second compound which is a curcumin and optionally a means to increase the water solubility and bioavailability of said second compound for use as a medicament.

Even more preferred the invention provides a pharmaceutical composition comprising a first compound which is hydroxychloroquine (HCQ), and a second compound which is a curcumin and whereby said means to increase the increase the water solubility and bioavailability of said second compound is the addition of a third compound piperine/BioPerine, for use as a medicament.

The invention further provides a pharmaceutical composition according to the invention for use in the treatment of a proliferative disorder/disease and/or a premalignant proliferative disorder/disease.

The invention further provides a pharmaceutical composition comprising a first compound which is a quinolinic compound or quinolinic like compound or a pharmaceutically acceptable salt thereof and a second compound which is a curcuminoid or curcuminoid like-compound for use in the treatment of a proliferative disorder/disease and/or a premalignant proliferative disorder/disease.

Even more preferred the invention provides a pharmaceutical composition comprising a first compound which is hydroxychloroquine (HCQ), and a second compound which is a curcumin for use in the treatment of a proliferative disorder/disease and/or a premalignant proliferative disorder/disease.

Even more preferred the invention provides a pharmaceutical composition comprising a first compound which is hydroxychloroquine (HCQ), and a second compound which is a curcumin and whereby said means to increase the increase the water solubility and bioavailability of said second compound is the addition of a third compound piperine/BioPerine, for use in the treatment of a proliferative disorder/disease and/or a premalignant proliferative disorder/disease.

The invention further provides a pharmaceutical composition comprising at least one first compound which is an autophagy and/or proteasome and/or Toll like receptor (TLR) modulator and a lysosomotropic agent, alone or in combination with at least one second compound which is an autophagy and/or proteasome and/or Toll-like receptor (TLR) modulator and a heat shock protein modulator, and optionally a means to increase the water solubility and bioavailability of said second compound for use in the prevention or treatment of a proliferative disorder and/or a premalignant proliferative disorder and/or to cause a regression of the proliferative disease.

The invention further provides a pharmaceutical composition according to the invention comprising at least a first compound (one or more) which is an autophagy and/or proteasome and/or Toll like receptor (TLR) modulator and a lysosomotropic agent alone for use in the prevention or treatment of a proliferative disorder and/or a premalignant proliferative disorder, more specifically to reduce/eliminate the abnormal cell clone (the "quiescent" and/or "non quiescent" abnormal cell clone) of a proliferative disorder, preferably a premalignant proliferative disorder, and to cause a regression of the proliferative disease.

The invention further provides a pharmaceutical composition according to the invention comprising at least a first compound (one or more) which is a quinolinic compound or quinolinic like compound or a pharmaceutically acceptable salt thereof alone or in combination with a second compound which is a curcuminoid or curcuminoid like-compound, for use in the prevention or treatment of a proliferative disorder and/or a premalignant proliferative disorder.

Said first compound is considered to be the main pharmacological active component of the composition. Said first compound a quinolinic compound or quinolinic like compound can act alone but is more effective in combination with a second compound a curcuminoid or curcuminoid like compound and/or a third compound a pyridine-piperidine group of alkaloids to reduce/eliminate the abnormal cell clone (the "quiescent" and/or "non quiescent" abnormal cell clone) of a proliferative disorder, preferably a premalignant proliferative disorder, and to cause a significant regression of the proliferative disease. For example the abnormal cell clone of a plasma cell and/or lymphocyte proliferative disorder. Preferably a B- lymphocyte proliferative disorder.

The compounds of the pharmaceutical composition of the invention are included in an amount sufficient to enhance the activity of each other, such that the two (or more) compounds together have greater therapeutic efficacy than the individual compounds given alone (e.g., due to synergistic interaction; reduced combined toxicity etc.).

Plasma cell disorders are well known in the art and include plasma cell neoplasms, plasma cell dyscrasias, Plasma cell myeloma, Plasmacytoma, Monoclonal immunoglobulin deposition diseases (amyloidosis), Heavy chain diseases, Extranodal marginal zone B cell lymphoma, also called MALT, lymphoma, Nodal marginal zone B cell lymphoma (NMZL), Follicular lymphoma, Mantle cell lymphoma, Diffuse large B cell lymphoma, Mediastinal (thymic) large B cell lymphoma, Intravascular large B cell lymphoma, Primary effusion lymphoma, Burkitt lymphoma/leukemia.

Included are neoplastic diseases involving proliferation of a single clone of cells producing a serum M component (a monoclonal immunoglobulin or immunoglobulin fragment); the cells usually have plasma cell morphology, but may have lymphocytic or lymphoplasmacytic morphology; this group includes multiple myeloma, Waldenström's macroglobulinemia, the heavy chain diseases, benign monoclonal gammopathy, and immunocytic amyloidosis. These neoplastic diseases are also called dysproteinemias , monoclonal gammopathies or monoclonal immunoglobulinopathies , and paraproteinemias.

The invention further provides the use of a pharmaceutical composition according to the invention for the preparation of a medicament for the treatment of a proliferative disorder and/or a premalignant proliferative disorder.

The invention further provides a pharmaceutical composition comprising at least a first compound according to the invention, alone, or in combination with a second compound according to the invention, and optionally a third compound according to the invention as a means to increase the water solubility of said second compound for use in the prevention or treatment of a proliferative disorder and/or a premalignant proliferative disorder.

The invention further provides a method of treating a proliferative disorder and/or a premalignant proliferative disorder/disease, in a subject, comprising administering to the subject effective amount of a pharmaceutical composition according to the invention.

The invention further provides a pharmaceutical composition according to the invention for use in the prevention or treatment of a premalignant proliferative disorder and/or malignant proliferative disease/disorder.

The invention further provides use of a pharmaceutical composition according to the invention for the manufacture of a medicament for the prevention or treatment of a premalignant proliferative disorder and/or a malignant proliferative disorder/disease.

For example, but not limited to a premalignant plasma cell and/or lymphocyte proliferative disorder. To reduce or preferably eliminate the abnormal cell clone of a premalignant proliferative disorder. By way of example to reduce or eliminate the abnormal cell clone of a B- lymphocyte premalignant proliferative disorder and/or the abnormal cell clone of a premalignant plasma cell proliferative disorder MGUS and/or SMM and/or IMM or the like to cause a regression of the disease.

The invention further provides a pharmaceutical composition according to the invention for use the treatment of a premalignant or malignant proliferative disease dependant on the heat shock response. For example, a proliferative disease dependant on Hsp90 and/or Hsp70 and/or Hsc70 (constitutive heat shock 70 protein) protein response.

In a preferred embodiment is a pharmaceutical composition according to the invention for use in the prevention or treatment of a proliferative disorder. More preferably to reduce and/or eliminate the abnormal cell clone of a proliferative disorder, a premalignant (benign, precancerous) or malignant (cancerous) proliferative disorder. In another preferred embodiment said proliferative disorder is a premalignant plasma cell proliferative disorder SMM, IMM and/or MGUS.

As used herein, "treatment" of a proliferative disorder including hyper-proliferative disorder refers to methods of killing, inhibiting or slowing the growth or increase in size of a body or population of proliferative cells (abnormal cell clone) or tumor or pre-cancerous growth, reducing proliferative cell numbers, or preventing spread to other anatomic sites, as well as reducing the size of a proliferative growth or numbers of proliferative cells. As used herein, "treatment" is not necessarily meant to imply cure or complete abolition of proliferative growths.

As used herein, a treatment effective amount is an amount effective to result in the killing, the slowing of the rate of growth of abnormal proliferative cells, the decrease in size of a body of abnormal proliferative cells, and/or the reduction in number of abnormal proliferative cells and/or preferably elimination of abnormal proliferative cells. For example but not limited to the reduction or elimination of the premalignant abnormal plasma cell clone (for example the abnormal blood marrow plasma cell) more specifically in a plasma cell disorder - MM or SMM or MGUS subject to at least cause a disease regression.

The invention further provides a pharmaceutical composition according to the invention for use to reduce and/or eliminate the "quiescent" and/or "non quiescent" abnormal cell clone of a proliferative disorder and/or a premalignant proliferative disorder. More in particular the invention provides a composition according to the invention (for use) to reduce or eliminate the "quiescent cells" and "non-quiescent" abnormal cell clone of a proliferative disorder/disease.

More in particular (for use) to reduce or eliminate the "quiescent cells" and "non-quiescent" abnormal cell clone of a premalignant proliferative disorder.

It is understood that the abnormal cell clone of a premalignant proliferative disorder comprises both "quiescent cells" and "non-quiescent cells". Quiescent cells as used herein refer to cells which preferably have a low level of mutations and little or noNF-κB activation, which may also be translationally attenuated, or cells that do not have the required specific mutation(s) more in particular, not angiogenesis primed and/or autophagy suppressed and/or proteasome inhibited for triggering sufficient NF-κB activation to facilitate angiogenesis. Preferably these cells are relatively quiescent (i.e., low proliferation rate)) sufficient to enter a quiescent state. Quiescence state as used herein is a temporary, reversible reduction in cell division/differentiation (i.e. cells become senescent and stop dividing or dormant/inactive) or total absence of proliferation (Pajalunga D et al., 2007; incorporated herein by reference). That is the dormant-to-active switch in the cells is in the 'off' position.

Quiescent cells are resting/quiet/dormant (have a temporary cessation of activity) but might be stimulated later to divide and proliferate to become non-quiescent cells, for example malignant/proliferating cancerous cells. Non quiescent cells are functioning cells mitotically active (dividing) cells. Preferably said second compound can inhibit quiescent cells malignant progression through heat shock protein inhibition and/or telomerase activity and/or induction of apoptosis.

By way of illustration of the present invention the combined reduction in TLR signaling (TRL-9 and TRL-4 inhibition) of a pharmaceutical composition of the present invention may result in NF-κB inactivation which inhibits TLR activated apoptosis (TRAIL - TNF related apoptosis-inducing ligand (CD253) resistance in a premalignant or malignant abnormal cell clone of a proliferative disorder. In addition a composition of the present invention is surprisingly efficient in blocking cellular cytotoxic stress survival pathways, amongst others the heat shock response (HSR) pathway giving rise to the unexpected observed reduction as disclosed herein the invention of the abnormal plasma cell clone of a proliferative disorder. More in particular a premalignant proliferative disorder and the regression of a proliferative disease from a near MM state, SMM or IMM state to a MGUS state through reduction/elimination/apoptosis of the abnormal plasma cell clone.

With the appropriate cellular trigger a "quiescent" premalignant abnormal (plasma) cell clone can be altered into a "non-quiescent" malignant abnormal (plasma) cell clone. For example but not limited to the differences between myeloma states resides in the frequency, type and number of mutations that can trigger NF-κB activation thus angiogenesis that is the conversion through specific mutations of quiescent cells into non-quiescent angiogenesis primed cells.

A pharmaceutical composition of the present invention is surprisingly effective against the quiescent and/or partially quiescent and/or non quiescent cells of the abnormal cell clone of a proliferative disorder. More in particular, against the translationally attenuated, mutated but non proliferating premalignant plasma cell clone. More in particular, against the (quiescent and/or partially quiescent and/or non quiescent) abnormal cell clone of monoclonal gammopathy of undetermined significance (MGUS) cell and/or smoldering (asymptomatic) multiple myeloma (SMM) and/or Indolent multiple myeloma (IMM) cells. A pharmaceutical composition of the present invention can surprisingly interfere with and/or block all major adaptive pro-survival pathway of (by way of example) the abnormal "quiescent" premalignant plasma cell clone of SMM and/or MGUS in addition to "non quiescent" malignant cancerous cells like the malignant plasma cell clone of MM. Surprisingly a pharmaceutical composition of the present invention through sustained use can effectively prevent more in particular the "quiescent" abnormal cell clone of a proliferative disorder developing resistance to therapy and survival for example through the use of cytotoxic stress survival pathways and mutations which has been noted with the use of prior art compositions and treatments, giving rise to the observed surprising reduction in and/or elimination of the abnormal plasma cell clone of a proliferative disorder. For example the observed regression of a plasma cell disorder from a near MM state, SMM or IMM state to a MGUS state through reduction/elimination of the abnormal "quiescent" and/or "non quiescent" plasma cell clone. By way of illustration but not limited to it may interfere with the heat shock response (HSR) through inhibition of the expression of HSR chaperones, like HSP90 and associated client proteins thereby destabilizing the proteasome. Proteasomes are part of a major mechanism by which cells regulate the concentration of particular proteins and degrade misfolded proteins.

Surprisingly this reduction in the abnormal plasma cell clone of proliferative disorder was independent of a reduction in Ig serum monoclonal component. Other investigators found serum paraprotein levels to be a factor influencing the probability of malignant conversion. On the contrary, as disclosed herein the invention, the multivariate model excluded a significant relation of serum MC size with disease progression. Thus unexpectedly confirming that the M-Ig count (monoclonal immunoglobulin), a commonly employed indicator of the disease status of plasma cell proliferative disorders, more in particular MGUS, SMM, or MM is in fact not a reliable indicator of disease status as previously thought.

In general the compositions described herein may be prepared in a conventional manner using conventional excipients or carriers that are well known in the art such as (solid) diluents, buffers, flavouring agents, binders, surface active agents, thickeners, lubricants, preservatives, xanthan gum, locust bean gum, galactose, other saccharides, oligosaccharides and/or polysaccharides, starch, starch fragments, dextrins, and the like and mixtures thereof. Preferably, the pharmaceutically acceptable carrier is of natural origin. The pharmaceutically acceptable carrier can further comprise an inert saccharide diluent selected from a monosaccharide or disaccharide.

It is an object of the invention to provide a composition according to the invention for use to slow the progression of and/or or to cause regression of a premalignant plasma cell proliferative disorder like (for example but not limited to) a monoclonal gammopathy of undetermined significance (MGUS) and/or smoldering (asymptomatic) multiple myeloma (SMM) and/or Indolent multiple myeloma (IMM) and/or to cause remission of a cancer arising from a premalignant plasma cell proliferative disorder like multiple myeloma (MM). Preferably said regression of a premalignant plasma cell proliferative disorder is a regression from a near MM state, SMM or IMM state to a MGUS state to at least reduce, preferably eliminate the abnormal cell clone of a proliferative disorder, like abnormal bone marrow plasma cells of myelopoliferative disorders (MPDs). All MPDs arise from precursors of the myeloid lineage in the bone marrow. The lymphoid lineage may produce similar diseases, the lymphoproliferative disorders (acute lymphoblastic leukemia, lymphomas, chronic lymphocytic leukemia and multiple myeloma

A proliferative disorder is one in which too many of a certain type of cell type, abnormal proliferation (production) i.e. overproduction of a certain cell type like for example lymphocytes or plasma cells, that are associated with autoimmune and/or an immunoglobulin disorders but not limited to. Examples of proliferative disorders are by way of example immunoproliferative disorders, immunoproliferative neoplasms, hypergammaglobulinemia, paraproteinemia, Mature T cell and natural killer (NK) cell neoplasms and the like.

Paraproteinemia, or monoclonal gammopathy, is the presence of excessive amounts of a single monoclonal gammaglobulin (in this case denominated "paraprotein") in the blood. It denotes an underlying immunoproliferative disorder. Included herein are disease related to paraproteinemias is Polyneuropathies (Polyneuropathy), Multiple Myeloma, Amyloidosis or Pemphigus (Pemphigus Vulgaris) and the like.

Preferably said disorder/disease presents with a monoclonal gammopathy or paraproteinemias or is a disease related to paraproteinemias or a polyclonal gammopathy. Preferably said disease is characterized by an unbalanced or disproportionate proliferation of immunoglobulin-producing cells, usually from a single clone. Preferably said disorder/disease is associated with an IgG paraproteins, a monoclonal or M-protein. These cells frequently secrete a structurally homogeneous immunoglobulin (M-component) and/or an abnormal immunoglobulin. For example Paraproteinemia, Plasma Cell Dyscrasias, Plasma Cell; Gammopathies, Paraimmunoglobulinemia but not limited to. However, approximately 1% of multiple myelomas are called nonsecretors because they do not produce any abnormal Ig.

The present invention concerns a pharmaceutical composition according to the invention for use in the prevention of a proliferative disorder and/or a premalignant proliferative disorder in a subject presenting with a immunoglobulin disorder and/or an autoimmune disorder and/or a inflammatory disorder and/or a nervous system disorder or in an asymptomatic subject at risk of developing these disorders. Preferably a premalignant proliferative disorder.

The present invention also concerns a pharmaceutical composition according to the invention for use in the prevention or treatment of and/or to reduce symptoms in a subject presenting with a proliferative disorder and/or an immunoglobulin disorder and/or an autoimmune disorder and/or an inflammatory disorder and/or a nervous system disorder or in an asymptomatic subject at risk of developing these disorders.

More in particular to prevent the development of a plasma cell and/or lymphocyte proliferative disorder in a subject presenting with an immunoglobulin disorder or an autoimmune disorder or an inflammatory disorder or a nervous system disorder or asymptomatic for but at risk of developing these disorders.

Examples of these disorders are well known in the art. For example but not limited to a malignant or non malignant B cell disorder - mature (monoclonal) B cell neoplasms, chronic lymphocytic leukemia/small lymphocytic lymphoma, B-cell prolymphocytic leukemia, Lymphoplasmacytic lymphoma (such as Waldenström macroglobulinemia) and the like or plasma cell disorder - plasma cell neoplasms, plasma cell myeloma plasmacytoma and the like.

By way of illustration a subject presenting with an autoimmune disease, more in particular in an asymptomatic subject with abnormal autoantibody levels thus at risk for experiencing one or more symptoms of a proliferative disorder more in particular a malignant B cell disorder or a plasma cell disorder.

As used herein an "autoantibody" is an antibody produced by a subject that binds to a self-antigen also produced by the subject. A composition of the present invention can be administered to a subject in an amount which prevents the subject (prophylactic treatment) from developing a proliferative disorder like a clonal disorder of plasma cells or lymphocytes associated with both malignant and non malignant disorders, often preceded or presenting with a monoclonal gammopathy for example a premalignant state called monoclonal gammopathy of undetermined significance (MGUS).

An "autoimmune disease/immunoglobulin disorder" herein is a disease or disorder arising from and directed against an individual's own tissues or a co-segregate or manifestation thereof or resulting condition therefrom. Examples of autoimmune diseases or disorders are well known in the art and include, but are not limited to Sjogren's syndrome, systemic lupus erythematosus (SLE), anti-phospholipid antibody syndrome, multiple sclerosis, ulcerative colitis, Crohn's disease, rheumatoid arthritis, Guillain-Barre syndrome, myasthenia gravis, large vessel vasculitis, medium vessel vasculitis, polyarteritis nodosa, pemphigus, scleroderma, Goodpasture's syndrome, glomerulonephritis, primary biliary cirrhosis, Grave's disease, membranous nephropathy, autoimmune hepatitis, celiac sprue, Addison's disease, polymyositis/dermatomyositis, monoclonal gammopathy, Factor VIII deficiency, cryoglobulinemia, peripheral neuropathy, IgM polyneuropathy, chronic neuropathy, and Hashimoto's thyroiditis, multiple sclerosis, autoimmune Bulbous Disease, Bullous pemphigoid and the like.

An asymptomatic subject is a carrier for a disease or infection but experiences no symptoms of the disease so is clinically silent for the disease or infection.

A "symptom" of a disease is any morbid phenomenon or departure from the normal in structure, function, or sensation, experienced by the subject and indicative of disease. For the purposes herein, a subject who is "at risk" for experiencing one or more symptoms of a proliferative disorder (clonal cell disorder) is by way of illustration a subject symptomatic or asymptomatic for an autoimmune disease, who presents with "abnormal" autoantibody levels. That is a concentration of autoantibody that exceeds the concentration of autoantibody present in a normal subject who is then at higher risk of developing a proliferating disorder (for experiencing symptoms of a malignant B cell disorder or plasma cell disorder) or an autoimmune disease if asymptomatic.

Prominent examples of inflammatory disorders are allergies, atherosclerosis, cancer (e.g., gallbladder carcinoma) and include auto-immune disorders include Coeliac disease, diabetes mellitus type 1 (IDDM), systemic lupus erythematosus (SLE), Sjögren's syndrome, Churg-Strauss Syndrome, Hashimoto's thyroiditis, Graves' disease, idiopathic thrombocytopenic purpura, and rheumatoid arthritis (RA) and the like. Immunoglobulin disorders more in particular immunoproliferative immunoglobulin disorders prominent examples include Plasmacytoma · Multiple myeloma (Plasma cell leukemia) · MGUS · *IgM* (Macroglobulinemia/Waldenström's macroglobulinemia) · heavy chain (Heavy chain disease) · light chain (Primary amyloidosis) and the like. Nervous system disorders include Immunological and inflammatory disorders of the central nervous system the brain and spinal cord like - Encephalitis, Meningitis, Tropical spastic paraparesis, Arachnoid cysts, Huntington's, Alzheimer's, Locked-in syndrome, Parkinson's, Tourette's, Multiple sclerosis and the like.

A subject is any vertebrate animal, preferably a mammal, including humans. Subjects to be treated by the methods of the present invention include both human subjects and animal subjects for veterinary purposes, for example dogs, cats and the like.

The invention provides a pharmaceutical composition according to the invention for use as a medicament.

The invention provides a pharmaceutical composition according to the invention for use as a medicament for the treatment of a proliferative disorder/disease and/or a premalignant proliferative disorder/disease.

The invention provides a pharmaceutical composition according to the invention for use as a medicament to slow the progression of and/or or to cause regression of a premalignant plasma cell proliferative disorder like a monoclonal gammopathy of undetermined significance (MGUS) and/or smoldering (asymptomatic) multiple myeloma (SMM) and/or Indolent multiple myeloma (IMM) and/or to cause remission of a cancer arising from a premalignant plasma cell proliferative disorder like multiple myeloma (MM).

The invention provides a pharmaceutical composition according to the invention for use as a medicament for the prevention of a proliferative disorder and/or a premalignant proliferative disorder in a subject presenting with a immunoglobulin disorder and/or an autoimmune disorder and/or a inflammatory disorder and/or a nervous system disorder or in an asymptomatic subject at risk of developing these disorders.

The invention provides a pharmaceutical composition according to the invention for use as a medicament for the prevention or treatment of and/or to reduce symptoms in a subject presenting with a proliferative disorder and/or an immunoglobulin disorder and/or an autoimmune disorder and/or an inflammatory disorder and/or a nervous system disorder or in an asymptomatic subject at risk of developing these disorders.

The invention further provides use of a pharmaceutical composition according to the invention in the preparation of a medicament for the treatment of a proliferative disorder/disease and/or a premalignant proliferative disorder/disease.

The invention further provides use of a pharmaceutical composition according to the invention for the manufacture of a medicament to slow the progression of and/or or to cause regression of a premalignant plasma cell proliferative disorder like a monoclonal gammopathy of undetermined significance (MGUS) and/or smoldering (asymptomatic) multiple myeloma (SMM) and/or Indolent multiple myeloma (IMM) and/or to cause remission of a cancer arising from a premalignant plasma cell proliferative disorder like multiple myeloma (MM).

The invention further provides use of a pharmaceutical composition according to the invention for the manufacture of a medicament for the prevention of a proliferative disorder and/or a premalignant proliferative disorder in a subject presenting with a immunoglobulin disorder and/or an autoimmune disorder and/or a inflammatory disorder and/or a nervous system disorder or in an asymptomatic subject at risk of developing these disorders.

The invention further provides use of a pharmaceutical composition according to the invention for the manufacture of a medicament for the prevention or treatment of and/or to reduce symptoms in a subject presenting with a proliferative disorder and/or an immunoglobulin disorder and/or an autoimmune disorder and/or an inflammatory disorder and/or a nervous system disorder or in an asymptomatic subject at risk of developing these disorders.

The invention further provides a method means and pharmaceutical composition according to the invention for use to eliminate abnormal cell clone of a proliferative disorder in a sample. By way of illustration to eliminate proliferative plasma cells and/or B-cells in donor blood samples of a subject, before transfusion. As used herein a sample is any body tissue that could comprise an abnormal proliferative cell clone - malignant B cells, plasma cell - like a blood sample (e.g. donor blood), a bone marrow sample (e.g. bone marrow sample) as a way of example but not limited to.

It is an object of the invention to provide a pharmaceutical composition according to the invention, wherein the HCQ content ranges from 200-1000 mg of HCQ, the curcumin content ranges from 1500mg - 12000mg and the piperine/BioPerine content ranges from 1-20mg. It has been shown that the risk of toxicity increases sharply toward 1% after 5 to 7 years of use, or a cumulative dose of 1000 g, of HCQ. More preferable HCQ, curcumin and BioPerine can be administered in a a pharmacologically effective amount, or therapeutically effective amount or simply effective amount which can be determined by one skilled in the art that is effective to produce the intended pharmacological, therapeutic or preventive result as disclosed herein the invention. More specifically to reduce and/or eliminate the abnormal proliferative cell clone, more specifically both the premalignant for example "a quiescent cell clone" and malignant "mutated and non- translationally attenuated" abnormal cell clone.

For example, if a given clinical treatment is considered effective when there is at least a 25% reduction in a measurable parameter associated with a disease or disorder, at least between 25-50% reduction of the abnormal cell clone of a proliferative disorder, that is a therapeutically effective amount of a drug for the treatment of that disease of disorder is the amount necessary to effect that at least a 25-50% reduction. Under certain circumstances, however, higher or lower daily doses may be appropriate depending on the ideal body weight of the subject to avoid over dosage that which can be determined by one skilled in the art. The administration of the daily dose can be carried out both by single administration in the form of an individual dose unit or else several smaller dose units and also by multiple administrations of subdivided doses at specific intervals. In a preferred embodiment HCQ is administered in a dose of 200-400mg daily and the curcumin - BioPerine/piperine preparation at a dosage of 2g/day on the basis of ideal body weight to avoid overdosage.

Amounts effective for therapeutic use (dose units) will, of course, depend on the stage of the disease, for example the abnormal blood plasma cell count, the activity of the compounds, manner of administration, age, weight and general state of the subject. Typically, dosages used in vitro can provide useful guidance in the amounts useful for in situ administration of the pharmaceutical composition. Animal models well known to those of skill in the art can be used to determine (daily) effective dosages for treatment of cancerous disorders, such as plasma cell disorders. Various considerations are described, e.g., in Gilman et al., eds. Goodman and Gilman's: The Pharmacological Bases of Therapeutics. 8th ed., Pergamon to Press, 1990; and Reminaton's Pharmaceutical Sciences, 17th ed., Mack Publishing Co. Easton, Pa., 1990, each of which is incorporated herein by reference. The pharmaceutical compositions are suitable for use in a variety of drug delivery systems to improve biological half life of the compounds for example liposomes, nanoparticules and other modern delivery vehicles known in the art.

It is an object of the present invention to provide a pharmaceutical composition as disclosed herein in a formulation for its intended route of administration. Administering the pharmaceutical composition of the present invention can be accomplished by various means known to a person skilled in the art. The pharmaceutical compositions according to the invention can be administered locally or systemically - intravenously orally or parenterally, or as implants, and even rectal use is possible in principle. It is understood that a therapeutically effective amount is administered. The term therapeutically effective amount or pharmacologically effective dose means the quantity of a compound according to the invention necessary to inhibit a symptom in a subject, that is the reduction or elimination of the premalignant abnormal plasma cell clone (for example the blood marrow plasma cell) more specifically in a plasma cell disorder - MM or SMM or MGUS subject. To slow the progression of and/or or to cause regression of and/or to eliminate the abnormal preferably premalignant plasma cell clone of a plasma cell disorder SMM and/or IMM and/or MGUS.

The pharmaceutically acceptable composition as disclosed herein the invention may be presented as an oral preparation. For example as a sustained release composition that can deliver the compounds in a desired manner for an extended period of time or as a controlled release composition. That is the therapeutically active agents are released from the composition at a controlled rate such that therapeutically beneficial blood levels (but below toxic levels) of the pharmaceutical composition/medicament are maintained over an extended period of time, e.g., providing a dosage form which provides effective levels of the medicament in-vivo for a time period of from about 1 to about 24 hours or more.

Suitable solid or liquid pharmaceutical preparation forms are, for example, granules, powders, tablets, coated tablets, cachet, (micro)capsules, suppositories, syrups, emulsions, suspensions, creams, aerosols, drops or injectable solution in ampule form and also preparations with protracted release of active compounds, in whose preparation excipients and additives and/or auxiliaries such as disintegrants, binders, coating agents, swelling agents, lubricants, flavorings, sweeteners or solublizers are customarily used and known in the art.

Commonly used pharmaceutical adjuvants or exipients or carriers which can be used as appropriate to formulate the composition for its intended route of administration like oral administration include inert excipients such as lactose, sodium carbonate, calcium phosphate or calcium carbonate, granulating and disintegrating agents such as corn starch or alginic acid; binding agents such as gelatin or starch; lubricating agents such as magnesium stearate, stearic acid or talc; preservative agents such as ethyl or propyl 4-hydroxybenzoate, and anti-oxidants, such as ascorbic acid. Tablet formulations may be uncoated or coated either to modify their disintegration and the subsequent absorption of the active ingredient within the gastrointestinal tract, or to improve their stability and/or appearance, in either case using conventional coating agents and procedures well known in the art. Pharmaceutically acceptable adjuvants and/or excipients for use in compositions known for the treatment of a cancer are well known in the art.

In general, pharmaceutically-acceptable salts include acid addition salts such as methanesulfonate, tosylate, α-glycerophosphate, fumarate, hydrochloride, citrate, maleate, tartrate and (less preferably) hydrobromide. Pharmaceutically-acceptable salts in general also include salts formed with phosphoric and sulfuric acid. Pharmaceutically-acceptable salts generally include base salts such as an alkali metal salt for example sodium, an alkaline earth metal salt for example calcium or magnesium, an organic amine salt for example triethylamine, morpholine, N-methylpiperidine, N-ethylpiperidine, procaine, dibenzylamine, N,N-dibenzylethylamine, tris-(2-hydroxyethyl)amine, tris (hydroxymethyl) methylammonium, N-methyl d-glucamine and amino acids such as lysine. There may be more than one cation or anion depending on the number of charged functions and the valency of the cations or anions.

The compounds of the present invention may be administered concurrent or in combination or simultaneous. The phrases "concurrent administration", "administration in combination", "simultaneous administration" or "administered simultaneously" as used herein, means that the compounds are administered at the same point in time or immediately following one another. In the latter case, the two compounds are administered at times sufficiently close that the results observed are indistinguishable from those achieved when the compounds are administered at the same point in time.

As used herein, the administration of two or more compounds "in combination" means that the two compounds are administered closely enough in time that the presence of one alters the biological effects of the other. The compounds may be administered simultaneously (concurrently) or sequentially. Where the administration is sequential or separate, the delay in administering the compounds should be such that each component should be present in the body so as to produce the therapeutic or pharmacologic or synergistic effect of the combination.

Simultaneous administration may be carried out by mixing the compounds prior to administration, or by administering the compounds at the same point in time but at different anatomic sites or using different routes of administration. Such a composition of the present invention conveniently provides the therapeutic combination of the invention for sequential or separate administration in the (synergistic) prevention or treatment of both a malignant but more specifically a premalignant plasma cell proliferative disorder, more in particular the reduction or elimination of abnormal bone marrow plasma cells. For example malignant or preferably abnormal premalignant/precancerous plasma cells (the plasma cell clone or monoclonal plasma cells) of the disorder but the separate compositions may also be administered simultaneously.

Administering" the pharmaceutical composition of the present invention can be accomplished by various means known to the skilled artisan. The pharmaceutical compositions according to the invention can be administered locally or systemically. The term "therapeutically effective amount" means the quantity of a compound according to the invention necessary to inhibit a symptom in a subject. A "subject" is any vertebrate animal, preferably a mammal, including humans. Subjects to be treated by the methods of the present invention include both human subjects and animal subjects for veterinary purposes. Animal subjects are preferably mammalian subjects including horses, cows, dogs, cats, rabbits, sheep, and the like.

It is a further object of the present invention to use a pharmaceutical composition according the invention in combination with other agents or therapies for use in the prevention of or treatment of a proliferative disorder (premalignant or malignant proliferative disorder/disease) in a subject asymptomatic or symptomatic for a proliferative disorder and/or an immunoglobulin disorder and/or an auto-immune disorder and/or an inflammatory disorder and/or a nervous system disorder.

For example by way of illustration but not limited;
1. Monoclonal antibody therapies such as the combination of lenalidomide with a monoclonal antibody elotuzumab.
2. Growth blockers drugs. Drugs designed to block the growth of proliferative cells by depriving them of required nutrient substances, through inhibition of the vascular endothelial growth factor (VEGF).
3. Other proteasome inhibitors- Bortezomib, carfilzomib and the like. Both competitive and non competitive proteasome inhibitors.
4. Immunomodulators - pomalidomide (Actimid) and the like.
5. Histone deacetylase (HDAC) inhibitors for example vorinostat (Zolinza) and panobinostat used alone or in combination with other proteasome inhibitors.
6. Akt inhibitors. For example the Akt inhibitor perifosine used alone or in combination with other proteasome inhibitors.
7. Other Heat shock protein-90 (Hsp90) inhibitors. For example alvespimycin and tanespimycin.
8. Rapamycin (mTOR) pathway inhibitors. For example the use of lenalidomide with an mTOR inhibitor everolimus (Afinitor) and the like alone or in combination with other proteasome inhibitors.
9. Cyclin-dependent kinase (CDK) inhibitors. For example flavopiridol and the like.
10. Telomerase inhibitors. For example imetelstat and the like.
11. RANK ligand inhibitors. For example denosumab to help prevent bone fractures and bone pain.
12. Piperlongumine (PL) [1-[(2*E*)-3-(3,4,5-Trimethoxyphenyl)prop-2-enoyl]-5,6-dihydropyridin-2(1*H*)-one] and its related analogs known in the art.
13. Thymoquinone (TQ).
14. Naltrexone.
15. Compounds with a piperonyl group.
16. Anti-cancer compounds known in the art. One of ordinary skill in the art is familiar with a variety of anti-cancer agents, or can find those agents in the routine art, which are used in the medical arts to treat cancer.

It is further an object of the present invention to provide a method for treating a subject asymptomatic (but at risk of developing said disorders) or symptomatic for a proliferative disorder and/or an immunoglobulin disorder and/or an auto-immune disorder and/or an inflammatory disorder and/or a nervous system disorder comprising administering a therapeutically effective amount of a pharmaceutical composition of the invention to a subject in need thereof.

It is further an object of the present invention to provide a method for treating a subject presenting with a proliferative disorder, and/or an immunoglobulin disorder and/or an autoimmune disorder and/or an inflammatory disorder and/or a nervous system disorder or in an asymptomatic subject at risk of developing these disorders comprising administering a therapeutically effective amount of a pharmaceutical composition comprising Hydroxychloroquine (HCQ), curcumin, and BioPerine or pharmaceutically acceptable salts thereof.

This method comprises administering a pharmaceutical composition of the invention to a subject preferably via the oral route to a mammal in need thereof, including a human, an amount which is effective to treat the disorder. The term "proliferative disorders" as used herein may be benign, premalignant or cancerous disorders and includes cancer, neoplastic growth, hyperplastic or proliferative growth or a pathological state of abnormal cellular development and includes solid tumors, non-solid tumors, and includes any abnormal cellular proliferation, such as that seen in leukemia or a plasma cell disorder. As used herein, "proliferative disorder" also means angiogenesis-dependent cancers and tumors, i.e., tumors that require for their growth (expansion in volume and/or mass) an increase in the number and density of the blood vessels supplying them with blood. Also included are Immunodeficiency-associated lymphoproliferative disorders, HIV and the like.

Proliferative disorder as used herein also includes neoplastic diseases of lymphoid tissues and blood and non-neoplastic diseases. By way of example the proliferative disease is a benign or malignant tumor, a carcinoma of the brain, kidney, liver, adrenal gland, bladder, breast, stomach, gastric tumors, ovaries, colon, rectum, prostate, pancreas, lung, vagina, thyroid, sarcoma, glioblastomas, multiple myeloma or gastrointestinal cancer, colon carcinoma or colorectal adenoma, or a tumor of the neck and head, an epidermal hyperproliferation, prostate hyperplasia, a neoplasia, or a leukemia.

A pharmaceutical composition of the present invention may be used to treat both neoplastic diseases and non-neoplastic diseases. Representative examples of non-neoplastic diseases are selected from the group consisting of psoriasis, benign proliferative skin diseases, ichthyosis, papilloma and the like.

Representative examples of neoplastic diseases are ovarian cancer, bladder cancer, respiratory tract, lung cancer, brain, cervical cancer, breast cancer, prostate cancer, gliomas, fibrosarcomas, retinoblastomas, melanomas, soft tissue sarcomas, reproductive organs, urinary tract, osteosarcomas, colon cancer, digestive tract, carcinoma of the kidney and pancreatic cancer, eye, liver, skin, head and neck, thyroid parathyroid and their distant metastases, but also includes lymphomas, sarcomas, and leukemias. Lymphomas include, but are not limited to AIDS-related lymphoma, non-Hodgkin's lymphoma, cutaneous T-cell lymphoma, Hodgkin's disease, and lymphoma of the central nervous system. These disorders have been well characterized in humans, but also exist with a similar etiology in other mammals, dogs, cats and the like and can be treated by administering the pharmaceutical compositions of the present invention.

Unless defined otherwise, all technical and scientific terms used herein have the same meaning as commonly understood by one of ordinary skill in the art. All references as mentioned herein are incorporated in their entirety. One of ordinary skill in the art will recognize that modifications and variations are possible without departing from the teachings of the invention. This description, and particularly the specific details of the exemplary embodiments disclosed, is provided primarily for clearness of understanding and no unnecessary limitations are to be understood therefrom, for modifications and other embodiments will become evident to those skilled in the art upon reading this disclosure and can be made without departing from the spirit or scope of the claimed invention.

### MATERIALS AND METHODS

### Patient X

Patient X is a woman of Afro-Surinamese descent age 51 and was diagnosed in November 2007 with Primary Sjögren's Syndrome Slumbering and Multiple Myeloma (SMM) type IgG kappa. In 2007 fluorescence in situ hybridization (FISH) was performed and no cytogenetic abnormalities were detected. X-ray skeletal overviews performed in 2007 and 2011 showed no abnormalities. Patient X takes a dose of 1 gram of curcumin/bioperin 3 times a day morning, noon and evening after meals and 200 mg of Plaquenil tablets (HCQ) once a day (at noon) after meals.

The patient undergoes a complete blood count (see below) every 6 months, a bone marrow plasma cell concentration (BMPC) determination (see below) every year, a complete skeletal X-ray overview every 3 or 4 years.

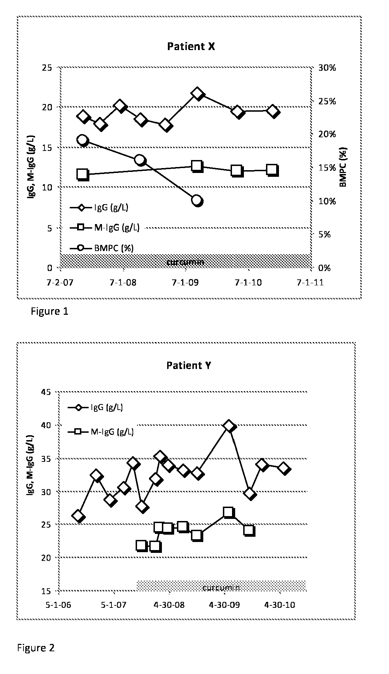

For the evolution of this patient's IgG, monoclonal IgG and BMPC, see figure 1, Table 1.

### Patient Y

Patient Y is a woman of Caucasian descent age 50 and was diagnosed in 1999 with MGUS (IgG, kappa). Patient Y takes a dose of 4 grams of curcumin/bioperin daily. Patient Y does not take HCQ.

The procedures for collecting data on this patient's IgG and monoclonal IgG is following current art and in general agreement with the ones described below for patient X.

Purely for illustration purposes and general information only; a data subset 1^{st} Nov 2005 - May 26 2010 concerning bone marrow plasma cell concentrations (BMPC), total (IgG) and monoclonal IgG (M-IgG) (updated regularly and cited in Margret's health blog) are incorporated herein by way of reference [see http://margaret.healthblogs.org].

For example Table 2 see: http://margaret.healthblogs.org
- test data 2007: http://margaret.healthblogs.org/life-with-myeloma/discovery-of-curcumin/my-curcumin-protocol/some-test-results/
- test data 2008: http://margaret.healthblogs.org/life-with-myeloma/discovery-of curcumin/my-curcumin-protocol/some-2008-test-results/
- test data 2009: http://margaret.healthblogs.org/life-with-myeloma/discovery-of-curcumin/my-curcumin-protocol/some-2009-test-results/
- test data 2010: http://margaret.healthblogs.org/life-with-myeloma/discovery-of-curcumin/my-curcumin-protocol/some-2010-test-results/
- treatment: http://margaret.healthblogs.org/life-with-myeloma/discovery-of-curcumin/my-curcumin-protocol/

For general information concerning the evolution of patient Y IgG and monoclonal IgG, see figure 2.

### Patient Z

Patient Z is a North-American male age 70 diagnosed with MGUS in mid 2003. His initial monoclonal IgG of 5.2 g/L (July 2) and BMPC of 9.8% (Aug 1) classify him as MGUS. The patient states "I have never felt any symptoms from my myeloma, only from the treatment. However, a PET scan in March, 2008, showed active regions of myeloma in both shoulder blades and one vertebral "spinous process," putting those bones at risk. " Patient Z took Curcumin 8000 mg to 14,000 mg daily in the period from June 27 to December 26, 2007, without any clear benefit. His m-spike did not react to treatment and his m-spike and BMPC two months later were so large that another treatment was decided. In the overlapping period from 17 Oct 2007 - March 7 2008, low dose naltrexone was taken, also without clear benefit. On March 7, 2008 a PET scan showed bone lesions and treatment was decided.

Purely for illustration purposes and general information only a data subset 17 Oct 2007 - March 7 2008 is cited from http://minnesotadon.ms11.net; incorporated herein by way of reference.

See
- test data: http://minnesotadon.ms11.net/Test%20Result%20Table.htm
- treatment data: http://minnesotadon.ms11.net/Treatment%20Table.htm

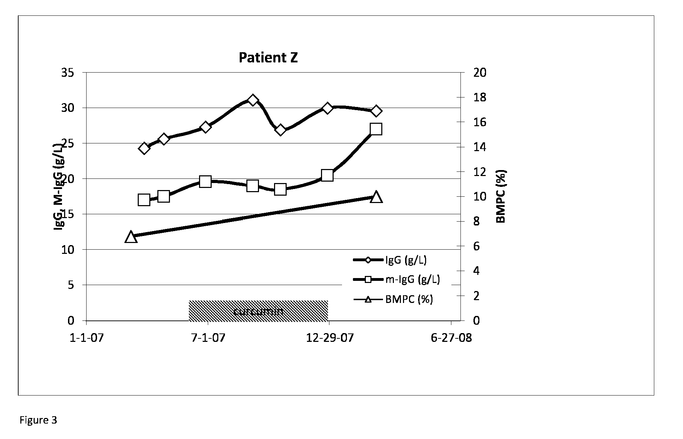

For general information concerning the evolution of patient's Z IgG, monoclonal IgG and BMPC, see figure 3 and table 3.

In this case curcumin did not prevent progression or cause a downward evolution of the BMPC nor the m-spike.

### Bone marrow plasma cell concentration (BMPC)

BMPC was determined on the basis of bone marrow aspirates (BMA) according to standard procedures known in the art [Stifter et al., 2010: Diagnostic Pathology 2010, Vol. 5, p. 30; incorporated herein by reference].

FISH cytogenetic analysis was performed once a year according to methods and procedures known in the art.
Frequency: once a year.

Complete blood count, specifically including determination of serum immunoglobulin content and monoclonal immunoglobulin fraction by immunoelectrophoresis was performed according to methods and procedures known in the art.
Frequency: twice a year.
Plaquenil® - hydroxychloroquine
Axemal(In India), Dolquine, or Quensyl

### Plaquenil ® tablets:

Plaquenil (hydroxychloroquine Sulfate) tablets contain 200 mg hydroxychloroquine Sulfate, equivalent to 155 mg base.
Dosage: 1 times daily 1 tablet of 200 mg. 6.5 mg / kg / day is considered safe for a non-obese person [see Marmor M et al., 2002: Ophthalmology 109, pp. 1377-1382; incorporated herein by reference and http://www.opt.indiana.edu/ce/plaq/risks.htm as a way of illustration and general information concerning Caring for the Plaquenil Patient.
Administration: oral.
Brand: Plaquenil (Sanofi Aventis)

### Description:

Hydroxychloroquine is a colorless crystalline solid, soluble in water to at least 20 %; chemically the drug is 2-[[4-[(7-Chloro-4-quinolyl) amino]pentyl]ethylamino] ethanol sulfate (1:1).

### Inactive Ingredients:

core: Lactose monohydrate, Povidone, Mais starch, Mg stearate
cover: Hypromellose, Macrogol 4000, TiO2, Lactose monohydrate

### Curcumin/BioPerine preparation.

Dosage: 3 times daily, 1 tablet of 1 gram.
Administration: oral.
Brand: Curcumin C3 complex (Doctor's Best Inc.)

### Description:

Turmeric root (Curcuma longa)
Standardized to >95% Curcuminoids 950 mg

### Including:

Bisdemethoxy Curcumin (2.5 - 6.5%)
Demethoxy Curcumin (15-25%)
Curcumin (70-80%) 1000 mg
Black Pepper Extract (Piper nigrum) (BioPerine^{®}) 5 mg
(piperine enhances the bioavailability of curcumin).

### Figure legends

### Figure 1.

Serum concentration of total (IgG) and monoclonal IgG (M-IgG) and bone marrow plasma cell concentration (BMPC) from patient X, who takes a dose of 1 gram of curcumin/bioperin 3 times a day and 200 mg of HCQ once a day. BMPC values as determined from bone marrow aspirate (BMA). BMPC values exhibit a striking, unexpected downward evolution, correlated with a lower risk of progression see table 4) while IgG and M-IgG remain constant. The grey bar indicates the period in which curcumin/piperine was taken. Unpublished original data.

**Table 1.**

| Serum concentration of total (IgG) and monoclonal IgG (M-IgG), bone marrow plasma cell concentration (BMPC) as determined from bone marrow aspirate (BMA), and treatment of patient X. | | | | |
|---|---|---|---|---|
| | IgG g/L | M-IgG g/L | BMPC % | Treatment |
| 11/9/07 | 18.9 | 11.5 | 19 | 3 gram of curcumin/bioperin + 200 mg of HCQ |
| 2/15/08 | 17.9 | | | |
| 6/9/08 | 20.3 | | | |
| 10/9/08 | 18.5 | | 16 | |
| 3/3/09 | 17.8 | | | |
| 9/4/09 | 21.7 | 12.6 | 10 | |
| 4/28/10 | 19.4 | 12.0 | | |
| 17/10/10 | 19.5 | 12.1 | | |

### Figure 2.

Serum concentration of total (IgG) and monoclonal IgG (M-IgG) of patiënt Y, who takes a dose of 4 grams of curcumin/bioperin daily but no HCQ. IgG, M-IgG values remain constant. The grey bar indicates the period in which curcumin/piperine was taken. For general information and illustrative purposes only a data subset is presented from Margret's health blog [see table 2 and http://margaret.healthblogs.org].

**Table 2.**

| Serum concentration of total (IgG) and monoclonal IgG (M-IgG) and treatment of patient Y. | | | |
|---|---|---|---|
| | IgG g/L | M-IgG g/L | Treatment |
| 1/11/05 | | | |
| 9/1/06 | 26.3 | | |
| 1/1/07 | 32.5 | | |
| 4/1/07 | 28.8 | | 4 grams of curcumin/bioperin daily but no HCQ |
| 7/1/07 | 30.6 | | |
| 9/1/07 | 34.3 | 20.7 | |
| 11/1/07 | 27.8 | 21.8 | |
| 1/31/08 | 31.9 | 21.7 | |
| 2/26/08 | 35.3 | 24.5 | |
| 4/23/08 | 34.0 | 24.4 | |
| 7/31/08 | 33.2 | 24.6 | |
| 11/1/08 | 32.8 | 23.3 | |
| 6/1/09 | 39.9 | 26.8 | |
| 10/13/09 | 29.7 | 24.1 | |
| 12/30/09 | 34.1 | | |
| 5/26/10 | 33.6 | | |

### Figure 3.

Serum concentration of total (IgG) and monoclonal IgG (M-IgG) and bone marrow plasma cell concentration (BMPC) of patient Z in the period from March 8, 2007 to March 7, 2008. The patient took curcumin with BioPerine 8000 - 14,000 mg curcumin from 6/27/07 until 12/26/07, but no HCQ. To this was added low dose naltrexone from 9/6/07 to 3/7/08. No clear benefit from the curcumin was seen in BMPC values. M-IgG serum concentration continued to rise and on March 7, 2008 treatment was decided after finding an increased bone marrow plasma cell concentration (table 3). BMPC values as determined from bone marrow aspirate (BMA). The grey bar indicates the period in which curcumin/piperine was taken. For general information and illustrative purposes only a data subset is presented from http://minnesotadon.ms11.net.

**Table 3.**

| Serum concentration of total (IgG) and monoclonal IgG (M-IgG), bone marrow plasma cell concentration (BMPC) as determined from bone marrow aspirate (BMA), and treatment of patient Z in the period from March 8, 2007 to March 7, 2008. | | | | |
|---|---|---|---|---|
| | IgG g/L | M-IgG g/L | BMPC % | Treatment |
| 3/8/07 | - | - | 6.8 | |
| 3/28/07 | 24.3 | 17 | - | |
| 4/26/07 | 25.6 | 17.5 | - | |
| 6/27/07 | 26.3 | 19.6 | - | CurcuminBioPerine 8g/d |
| 9/6/07 | 31.1 | 19.0 | - | CurcuminBioPerine 14g/d LDN, diet |
| 10/17/07 | 26.9 | 18.5 | - | Curcumin/BioPerine 14g/d LDN, diet |
| 12/26/07 | 30.0 | 20.5 | - | LDN, diet |
| 3/7/08 | 29.6 | 27 | 10% | |

**Table 4.**

| The number of progression incidences or "events" is significantly higher for SMM patients with BMPC>10% than with BMPC≤10% according to Cesana *et al*., 2002. Cesana et al 2002: Journal of Clinical Oncology, Vol 20, No 6: pp 1625-1634; Table 5, incorporated herein by reference as a means of illustration and as general information. Higher percentages are significantly associated with a worse clinical outcome. | | | | |
|---|---|---|---|---|
| BMPC | No. of events | Patients at risk | Event rate / 100 person years | 95 % CI |
| ≤ 10% | 17 | 108 | 2.5 | 1.6 - 4.0 |
| >10% | 8 | 19 | 11.3 | 5.0 - 22.5 |

### REFERENCES

Anand P et al., 2008. Biochem pharmacol 2008 Dec 1;76(11):1590-611
Baldini L et al., 1996; Blood. 1996 Feb 1;87(3):912-8
Bladé J and Rosiñol L., 2006. Curr Treat Options Oncol. 2006 May;7(3):237-45
Bladé J et al. 2009. Blood. 2009 May 28;113(22):5370
Cesana C et al 2002: Journal of Clinical Oncology, Vol 20, No 6: pp 1625-1634 Gilman et al., eds. Goodman and Gilman's: The Pharmacological Bases of Therapeutics. 8th ed., Pergamon to Press, 1990.
Gilmore TD. Multiple myeloma: lusting for NF-kappaB. Cancer Cell. 2007 Aug; 12(2):95-7.
Kapoor N et al. 2007. J Sci Ind Research 2007 August 66(8): 647-50
Kawanishi et al., Antioxid Redox Signal. 2005 Nov-Dec;7(11-12):1728-39
King RI and Florkowski CM, 2010; Pathology 42(5):397-401
Klionsky DJ et al., 2008. Autophagy. Feb 16;4(2):151-75
Kyle RA et al. 2010, Leukemia. 2010 Jun;24(6):1121-7.
Kyle RA et al., 2011. Clin Lymphoma Myeloma Leuk. Feb 1;11(1):74-6.
Landgren O, Kyle RA. Br J Haematol. 2007 Dec; 139(5):717-23.
Landgren O. et al., 2009. Blood 113(22):5412-7.
Marmor M et al., 2002: Ophthalmology 109, pp. 1377-1382
McMaster ML, Caporaso N, Br J Haematol. 2007 Dec; 139(5):663-71.
Morimoto S et al., 2000. J Immunol. 2000 Apr 15;164(8):4097-104
Pajalunga D et al., 2007. J Cell Biol. 2007 Mar 12;176(6):807-18
Rajkumar SV et al. 2010. Mayo Clin Proc. 85(10):945-948
Reminaton's Pharmaceutical Sciences, 1th ed., Mack Publishing Co. Easton, Pa., 1990
Shoba et al., 1998; Planta Med. 64 (4): 353-6.
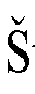 tifter et al 2010: Diagnostic Pathology 2010, Vol. 5, p. 30.
Teiten MH et al., 2010. Toxins, 2, 128-162
Stecher, VJ and Michner WF, 1997. Patent AU7549496, US55314894.
Vogel T et al., 2008. Blood (ASH Annual Meeting Abstracts) 2008 112: Abstract 3684
Weiss BM et al., 2009. Blood. 113(22):5418-22.

## Claims

1. A pharmaceutical composition comprising at least a first compound which is an autophagy and/or proteasome and/or Toll like receptor (TLR) modulator and a lysosomotropic agent, and at least a second compound which is an autophagy and/or proteasome and/or Toll-like receptor (TLR) modulator and a heat shock protein modulator and optionally a means to increase the water solubility and bioavailability of said second compound and optionally a pharmaceutically acceptable diluent or carrier.

2. A pharmaceutical composition according to claim 1 comprising at least a first compound which is a non-competitive proteasome inhibitor and a lysosomotropic agent and/or a second compound which is a non-competitive proteasome inhibitor which can modulate Hsp 90 function.

3. A pharmaceutical composition according to claim 1 or claim 2, whereby said first compound is a quinolinic compound or quinolinic like compound or a pharmaceutically acceptable salt thereof and whereby said second compound is a curcuminoid or curcuminoid like-compound or a pharmaceutically acceptable salt thereof and whereby said means to increase the water solubility and bioavailability of said second compound is the addition of at least one compound from a pyridine-*piperidine* group of *alkaloids* or a pharmaceutically acceptable salt thereof and optionally a pharmaceutically acceptable diluent or carrier.

4. A pharmaceutical composition according to any of claims 1 -3, whereby said first compound is an aminoquinoline an analog, derivative, or isomer thereof.

5. A pharmaceutical composition according to claim 4 whereby said first compound is selected from the group consisting of Hydroxychloroquine, Chloroquine, Amodiaquine Primaquine, Tafenoquine, Pamaquine, Mefloquine, Quinine, Quinidine, Piperaquine and Bisquinoline.

6. A pharmaceutical composition according to any of claims 1-5 whereby said first compound is Hydroxychloroquine, said second compound is curcumin and whereby said means to increase the increase the water solubility and bioavailability of said second compound is the addition of a third compound piperine/BioPerine.

7. A pharmaceutical composition comprising at least one first compound which is an autophagy and/or proteasome and/or Toll like receptor (TLR) modulator and a lysosomotropic agent and at least one second compound which is an autophagy and/or proteasome and/or Toll-like receptor (TLR) modulator and a heat shock protein modulator, and optionally a means to increase the water solubility and bioavailability of said second compound for use as a medicament.

8. A pharmaceutical composition comprising at least one first compound which is an autophagy and/or proteasome and/or Toll like receptor (TLR) modulator and a lysosomotropic agent, alone or in combination with at least one second compound compound which is an autophagy and/or proteasome and/or Toll-like receptor (TLR) modulator and a heat shock protein modulator, and optionally a means to increase the water solubility and bioavailability of said second compound for use in the prevention or treatment of a proliferative *disorder and*/*or* a premalignant proliferative disorder and/or *to cause a regression of the proliferative disease.*

9. A pharmaceutical composition according to any of claims 1-6, for use in the prevention or treatment of a proliferative disorder and/or a premalignant proliferative disorder.

10. A pharmaceutical composition according to any of claims 1-6, for use *to reduce and*/*or eliminate the "quiescent" andlor "non quiescent" abnormal cell clone of* a proliferative *disorder and*/*or* a premalignant proliferative disorder.

11. A pharmaceutical composition according to any of claims 1-6, for use to *slow the* progression of and/or or to cause *regression of a premalignant plasma cell* proliferative disorder *like* a monoclonal gammopathy of undetermined significance (MGUS) and/or smoldering (asymptomatic) multiple myeloma (SMM) and/or Indolent multiple myeloma (IMM) *and*/*or to cause remission of a cancer* arising from *a premalignant plasma cell* proliferative *disorder like* multiple myeloma (MM).

12. A pharmaceutical according to any of claims 1-6, for use in the prevention of a proliferative *disorder and*/*or* a premalignant proliferative disorder in a subject presenting with a immunoglobulin disorder and/or an autoimmune disorder and/or a inflammatory disorder and/or a nervous system disorder or in an asymptomatic subject at risk of developing these disorders.

13. A pharmaceutical according to any of claims 1-6, for use in the prevention or treatment of and/or to reduce symptoms in a subject presenting with a proliferative disorder and/or an immunoglobulin disorder and/or an autoimmune disorder and/or an inflammatory disorder and/or a nervous system disorder or in an asymptomatic subject at risk of developing these disorders.

14. The pharmaceutical composition according to anyone of claims 1-6, wherein the HCQ content ranges from 200-1000 mg of HCQ, the curcumin content ranges from 1500mg - 12000 mg and the Piperine/BioPerine content ranges from 1-20mg.

15. The pharmaceutical composition according to claim 14, in combination with other agents or therapies for use in the prevention of or treatment of a proliferative disorder in a subject asymptomatic or symptomatic for a proliferative disorder and/or an immunoglobulin disorder and/or an auto-immune disorder and/or an inflammatory disorder and/or a nervous system disorder.
